Europäisches Patentamt

European Patent Office

Office européen des brevets

(11) Publication number: **0 445 423 B1**

(12) **EUROPEAN PATENT SPECIFICATION**

(45) Date of publication of patent specification: **20.09.95**

(51) Int. Cl.6: **G01N 33/576**, C07K 14/00

(21) Application number: **90125354.2**

(22) Date of filing: **22.12.90**

(54) **Hepatitis C assay.**

(30) Priority: **22.12.89 US 456162**
**07.11.90 US 610180**

(43) Date of publication of application:
**11.09.91 Bulletin 91/37**

(45) Publication of the grant of the patent:
**20.09.95 Bulletin 95/38**

(84) Designated Contracting States:
**AT BE CH DE ES FR GB IT LI NL SE**

(56) References cited:
**EP-A- 0 318 216**
**EP-A- 0 388 232**

(73) Proprietor: **ABBOTT LABORATORIES**
**CHAD-0377,**
**AP6D/2,**
**One Abbott Park Road**
**Abbott Park,**
**Illinois 60064-3500 (US)**

(72) Inventor: **Leung, Tat**
**2825 Grandville Ct.,**
**Apt. 315**
**Waukegan, IL 60085 (US)**
Inventor: **Casey, James M.**
**3042 Grandville,**

**Apt. 211**
**Waukegan, IL 60085 (US)**
Inventor: **Sarin, Virender Kumar**
**516 Fairlawn Avenue**
**Libertyville, IL 60048 (US)**
Inventor: **Mehta, Smriti U.**
**1124 Kristin Drive**
**Libertyville, IL 60048 (US)**
Inventor: **Devare, Sushil G.**
**2492 Farnsworth Lane**
**Northbrook,**
**Illinois 60062 (US)**
Inventor: **Lesniewski, Richard R.**
**8706 110th Street**
**Kenosha, WI 53142 (US)**
Inventor: **Desai, Suresh M.**
**1408 Amy Lane**
**Libertyville, IL 60048 (US)**

(74) Representative: **Modiano, Guido, Dr.-Ing. et al**
**Modiano & Associati S.r.l.**
**Via Meravigli, 16**
**I-20123 Milano (IT)**

## Description

This invention relates generally to an assay for identifying the presence in a sample of an antibody which is immunologically reactive with a hepatitis C virus antigen and specifically to an assay for detecting a complex of an antibody and a polypeptide having at least one epitope of a hepatitis C virus antigen.

## BACKGROUND

Acute viral hepatitis is clinically diagnosed by a well-defined set of patient symptoms, including jaundice, hepatic tenderness, and an increase in the serum levels of alanine aminotransferase and aspartate aminotransferase. Additional serologic immunoassays are generally performed to diagnose the specific type of viral causative agent. Historically, patients presenting clinical hepatitis symptoms and not otherwise infected by hepatitis A, hepatitis B, Epstein-Barr or cytomegalovirus were clinically diagnosed as having non-A non-B hepatitis (NANBH) by default. The disease may result in chronic liver damage.

Each of the well-known, immunologically characterized hepatitis-inducing viruses, hepatitis A virus (HAV), hepatitis B virus (HBV), and hepatitis D virus (HDV) belongs to a separate family of viruses and has a distinctive viral organization, protein structure, and mode of replication.

Attempts to identify the NANBH virus by virtue of genomic similarity to one of the known hepatitis viruses have failed, suggesting that NANBH has a distinct organization and structure. [Fowler, et al., J. Med. Virol., 12:205-213 (1983) and Weiner, et al., J. Med. Virol., 21:239-247 (1987)].

Progress in developing assays to detect antibodies specific for NANBH has been particularly hampered by difficulties in correctly identifying antigens associated with NANBH. See, for example, Wands, J., et al., U.S. Patent 4,870,076, Wands, et al., Proc. Nat'l. Acad. Sci., 83:6608-6612 (1986), Ohori, et al., J. Med. Virol., 12:161-178 (1983), Bradley, et al., Proc. Nat'l. Acad. Sci., 84:6277-6281, (1987), Akatsuka, T., et al., J. Med. Virol , 20:43-56 (1986), Seto, B., et al., WO-A-90/02206, Takahashi, K., et al., European Patent Application No. 0 293 274, published November 30, 1988, and Seelig, R., et al., in PCT Application PCT/EP88/00123 (WO-A-88/06184).

Recently, another hepatitis-inducing virus has been unequivocally identified as hepatitis C virus (HCV) by Houghton, M., et al., European Patent Application publication number 0 318 216, May 31, 1989. Related papers describing this virus include Kuo, G., et al., Science, 244:359-361 (1989) and Choo, Q., et al., Science, 244:362-364 (1989). Houghton, M., et al. reported isolating cDNA sequences from HCV which encode antigens which react immunologically with antibodies present in patients infected with NANBH, thus establishing that HCV is the viral agent causing NANBH.

The cDNA sequences associated with HCV were isolated from a cDNA library prepared from the RNA obtained from pooled serum from a chimpanzee with chronic HCV infection. The cDNA library contained cDNA sequences of approximate mean size of about 200 base pairs. The cDNA library was screened for encoded epitopes expressed in clones that could bind to antibodies in sera from patients who had previously experienced NANBH.

In the European Patent Application 0 318 216, Houghton, M., et al. also described the preparation of several superoxide dismutase fusion polypeptides (SOD) and the use of these SOD fusion polypeptides to develop an HCV screening assay. The most complex SOD fusion polypeptide described in the European Patent Application, designated C100-3, was described as containing 154 amino acids of human SOD at the aminoterminus, 5 amino acid residues derived from the expression of a synthetic DNA adapter containing a restriction site, EcoRI, 363 amino acids derived from the expression of a cloned HCV cDNA fragment, and 5 carboxy terminal amino acids derived from an MS2 cloning vector nucleotide sequence. The DNA sequence encoding this polypeptide was transformed into yeast cells using a plasmid. The transformed cells were cultured and expressed a 54,000 molecular weight polypeptide which was purified to about 80% purity by differential extraction.

Other SOD fusion polypeptides designated SOD-NANB$_{5-1-1}$ and SOD-NANB$_{81}$ were expressed in recombinant bacteria. The E.coli fusion polypeptides were purified by differential extraction and by chromatography using anion and cation exchange columns. The purification procedures were able to produce SOD-NANB$_{5-1-1}$ as about 80% pure and SOD-NANB$_{81}$ as about 50% pure.

The recombinant SOD fusion polypeptides described by Houghton, M., et al. were coated on microtiter wells or polystyrene beads and used to assay serum samples. Briefly, coated microtiter wells were incubated with a sample in a diluent. After incubation, the microtiter wells were washed and then developed using either a radioactively labelled sheep anti-human antibody or a mouse antihuman IgG-HRP (horseradish peroxidase) conjugate. These assays were used to detect both post acute phase and chronic phase HCV infection. Due to the preparative methods, assay specificity required adding yeast or E.coli

2

EP 0 445 423 B1

extracts to the samples in order to prevent undesired immunological reactions with any yeast or E.coli antibodies present in samples.

Application EP-A-0 388 232 discloses further HCV sequences and polypeptides.

Ortho Diagnostic Systems Inc. have developed a research immunoenzyme assay to detect antibodies to HCV antigens. The Ortho assay procedure is a three-stage test for serum/plasma carried out in a microwell coated with the recombinant yeast/hepatitis C virus SOD fusion polypeptide C100-3.

In the first stage, a test specimen is diluted directly in the test well and incubated for a specified length of time. If antibodies to HCV antigens are present in the specimen, antigen-antibody complexes will be formed on the microwell surface. If no antibodies are present, complexes will not be formed and the unbound serum or plasma proteins will be removed in a washing step.

In the second stage, anti-human IgG murine monoclonal antibody horseradish peroxidase conjugate is added to the microwell. The conjugate binds specifically to the antibody portion of the antigen-antibody complexes. If antigen-antibody complexes are not present, the unbound conjugate will also be removed by a washing step.

In the third stage, an enzyme detection system composed of o-phenylenediamine 2HCl (OPD) and hydrogen peroxide is added to the test well. If bound conjugate is present, the OPD will be oxidized, resulting in a colored end product. After formation of the colored end product, dilute sulfuric acid is added to the microwell to stop the color-forming detection reaction.

The intensity of the colored end product is measured with a microwell reader. The assay may be used to screen patient serum and plasma.

It is established that HCV may be transmitted by contaminated blood and blood products. In transfused patients, as many as 10% will suffer from post-transfusion hepatitis. Of these, approximately 90% are the result of infections diagnosed as HCV. The prevention of transmission of HCV by blood and blood products requires reliable, sensitive and specific diagnosis and prognostic tools to identify HCV carriers as well as contaminated blood and blood products. Thus, there exists a need for an HCV assay which uses reliable and efficient reagents and methods to accurately detect the presence of HCV antibodies in samples.

BRIEF SUMMARY

The present invention provides an improved assay for detecting the presence of an antibody to an HCV antigen in a sample by contacting the sample with polypeptide containing at least one epitope of an HCV antigen.

One assay format according to the invention provides a confirmatory assay for unequivocally identifying the presence of an antibody that is immunologically reactive with an HCV antigen. Briefly, a fluid sample is used to prepare first and second aliquots. The aliquots are then contacted with at least two polypeptides duplicative of a continuous amino acid sequence putatively contained in proteins expressed by clones containing HCV cDNA sequences containing at least one epitope of an HCV antigen under conditions suitable for complexing the antibody with the polypeptide. Finally, the antibody-antigen complex is detected. The improvement comprises contacting the first aliquot with recombinant polypeptide C100-3, and contacting the second aliquot with one or more polypeptides selected from the group consisting of p1, p35, p99, p1192, p1223, p1684, p1689, p1694, p1866, p1899, p380, p380.LG, p447, p607, p643 b, p666, p691 and p2302 provided that assays in which the second aliquot is contacted with p1689 as the only polypeptide are excluded. Preferred polypeptides are selected from the group consisting of p1684, p1689, p1866, p380, p643b, p666, p2302 and p380.LG.

Another assay format provides a combination assay for detecting the presence of an antibody that is immunologically reactive with an HCV antigen in a fluid sample by contacting the sample with a polypeptide containing at least one epitope of an HCV antigen under conditions suitable for complexing the antibody with the polypeptide and detecting the antibody-polypeptide complex. The improvement comprises contacting the sample with a solid support containing commonly bound recombinant polypeptide C100-3 and a polypeptide selected from the group consisting of p1, p35, p99, p1192, p1223, p1684, p1694, p1866, p1899, p380, p380.LG, p447, p607, p643b, p666, p691 and p2302.

Another assay format provide, an assay for identifying the presence of an antibody that is immunologically reactive with an HCV antigen in a fluid sample comprising contacting the sample with a polypeptide containing at least one epitope of in HCV antigen selected from the group consisting of p1, p35, p99, p1192, p1223, p1684, p1694, p1866, p1899, p380, p380.LG, p447, p607, p643b, p666, p691 and p2302. under conditions suitable for complexing the antibody with the polypeptide and detecting the antibody-polypeptide complex.

3

Another assay format provides an immunodot assay for identifying the presence of an antibody that is immunologically reactive with an HCV antigen by concurrently contacting a sample with at least two polypeptides each containing distinct epitopes of an HCV antigen under conditions suitable for complexing the p1684, p1689, p1694, p1866, p1899, p380, p380.LG, p447, p607, p643b, p666, p691, p2302 and C100-3. The assay methods employing only p1689 and C100-3 are excluded. Preferred polypeptides are selected from the group consisting of p1684, p1694, p1684, p1866, p380, p643b, p666, p2302, p380.LG and C100-3.

Another assay format provides a competition assay directed to the confirmation that positive results are not false by identifying the presence of an antibody that is immunologically reactive with an HCV antigen in a fluid sample where the sample is used to prepare first and second immunologically equivalent aliquots. The first aliquot is contacted with solid support containing a bound polypeptide which contains at least one epitope of an HCV antigen under conditions suitable for complexing with the antibody to form a detectable antibody-polypeptide complex and the second aliquot is first contacted with unbound polypeptide and then contacted with the same, solid support containing bound polypeptide. The improvement comprises selecting the polypeptide from the group consisting or p1, p35, p99, p1192, p1223, p1684, p1694, p1866, p1899, p380, p380.LG, p447, p607, p643b, p666, p691 and p2302.

In all of the assays, the sample is diluted before contacting the polypeptide absorbed on a solid support. Samples may be obtained from different biological samples such as whole blood, serum, plasma, cerebral or spinal fluid, and lymphocyte or cell culture supernatants. Solid support materials may include cellulose materials, such as paper and nitrocellulose, natural and synthetic polymeric materials, such as polyacrylamide, polystyrene, and cotton, porous gels such is silica gel, agarose, dextran and gelatin, and inorganic materials such as deactivated alumina, magnesium sulfate and glass. Suitable solid support materials may be used in assays in a variety of wall known physical configurations, including microtiter wells, test tubes, beads, strips, membranes, and microparticles. A preferred solid support for a non-immunodot assay is a polystyrene bead. A preferred solid support for on immunodot assay is nitrocellulose.

Suitable methods and reagents for detacting an antibiody-antigen complex in an assay of the present invention are commercially available or known in the relevant art. Representative methods may employ detection reagents such as enzymatic, radioisotopic, fluorescent, luminescent, or chemiluminescent reagents. These reagents may be used to prepare hapten-labelled antihapten detection systems according to known procedures, for example, a biotin-labelled antibiotin system may be used to detect in antibody-antigen complex.

The present invention also encompasses assay kits including polypeptides which contain at least one epitope of an HCV antigen bound to a solid support as well as needed sample preparation reagents, wash reagents, detection reagents and signal producing reagents.

Other aspects and advantages of the invention will be apparent to those skilled in the art upon consideration of the following detailed description which provides illustrations of the invention in its presently preferred embodiments.

## DESCRIPTION OF THE DRAWINGS

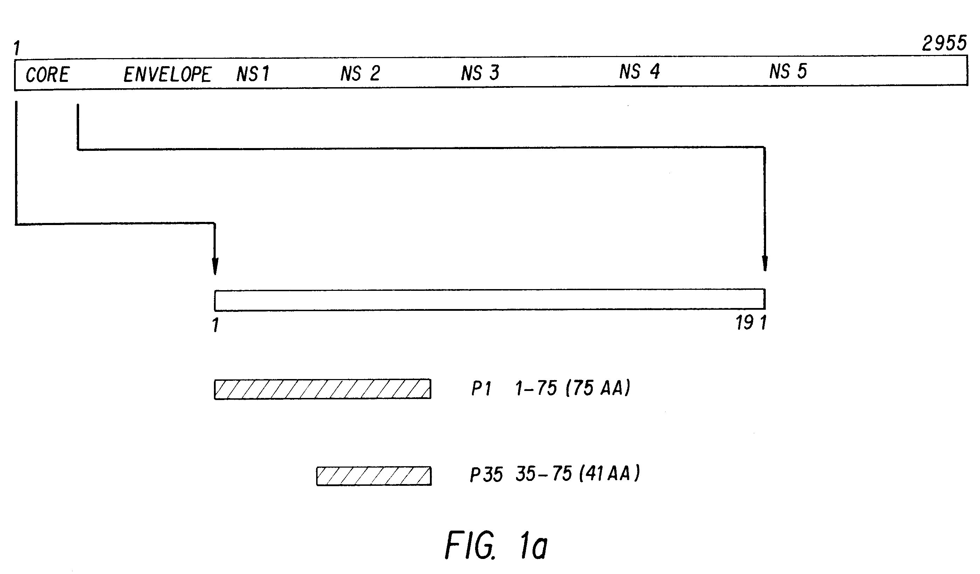

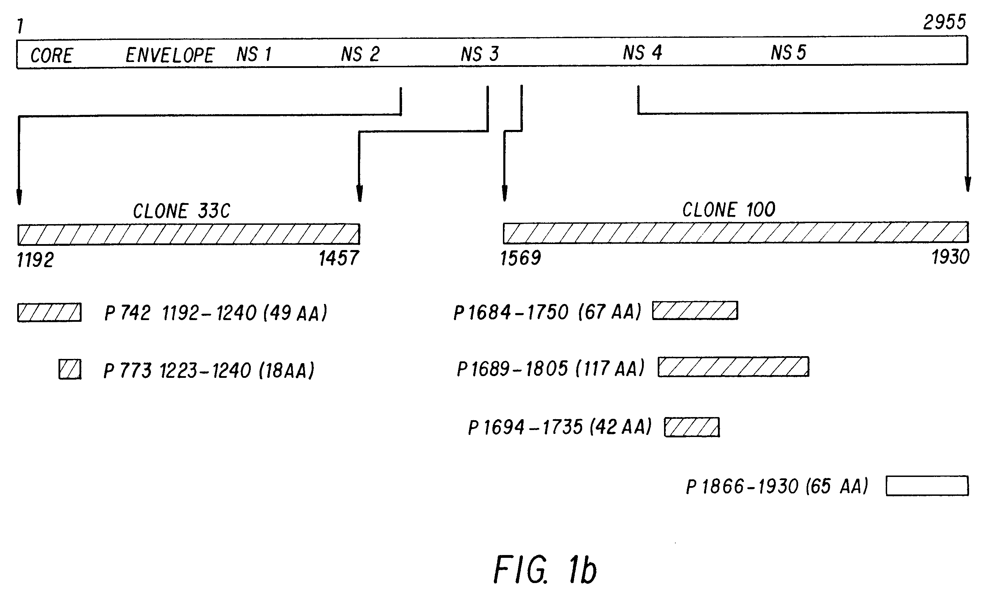

FIGURES 1a and 1b illustrate the HCV genome.

FIGURE 2 illustrates the use of antigenic polypeptides to identify the presence of antibodies in a chimpanzee inoculated with HCV.

FIGURES 3a and 3b illustrate the sensitivity increase using a combination assay format.

FIGURE 4 illustrates a test cartridge for an immunodot assay.

FIGURE 5 illustrates a seroconversion graph wherein the amount of anti-NS-5 S/N antibody, shown as the solid line between closed circles, and the amount of anti-HCV 2.0 S/CO antibody shown as a solid line between open squares, is plotted against days post presentation.

## DETAILED DESCRIPTION

The present invention is directed to an assay to detect an antibody to an HCV antigen in a sample. Human serum or plasma is diluted in a sample diluent and incubated with a polystyrene bead coated with a polypeptide that includes an HCV antigenic epitope. If antibodies are present in the sample they will form a complex with the antigenic polypeptide and become affixed to the polystyrene bead. After the complex has formed, unbound materials and reagents are removed by washing the bead and the bead-antigen-antibody complex is reacted with a solution containing horseradish peroxidase labeled goat antibodies directed against human antibodies. This peroxidase enzyme then binds to the antigen-antibody complex already fixed to the bead. In a final reaction the horseradish peroxidase is contacted with o-phenylenediamine and

4

hydrogen peroxide which results in a yellow-orange color. The intensity of the color is proportional to the amount of antibody which initially binds to the antigen fixed to the bead.

The preferred polypeptides having HCV antigenic epitopes were selected from portions of the HCV genome which encoded polypeptides which possessed amino acid sequences similar to other known immunologically reactive agents and which were identified as having some immunological reactivity. (The immunological reactivity of a polypeptide was initially identified by reacting the cellular extract of E. coli clones which had been transformed with cDNA fragments of the HCV genome with HCV infected serum. The clones presumably expressed polypeptides encoded by the incorporated cDNA which were immunologically reactive with serum known to contain antibody to HCV antigens.) An analysis of a given amino acid sequence, however, only provides rough guides to predicting immunological reactivity. There is no invariably predictable way to ensure immunological activity short of preparing a given amino acid sequence and testing the suspected sequence in an assay. As illustrated in Table 1, some peptides which were expected to provide immunological reactivity were found to be unreactive when used in an actual assay.

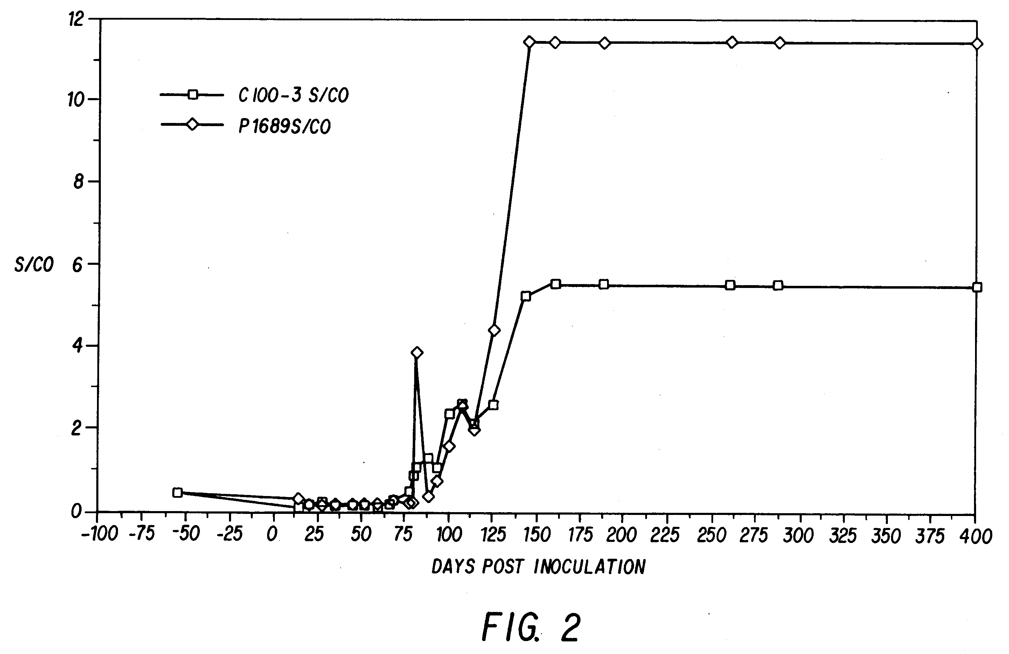

The use of polypeptides having one or more than one epitope of an HCV antigen to detect the presence of an antibody to an HCV antigen is illustrated in Figure 2. The course of HCV infection in the chimpanzee, Melilot, was followed with one assay using recombinant C100-3 polypeptide and with another assay using p1689 polypeptide. Both assays gave negative results before inoculation and both assays detected the presence of antibodies about 100 days after the animal had been infected with HCV.

It is to be noted though that assays depending on C100-3 or p1689 alone or only on the combination of C100-3 and p1689 are not part of the present invention.

## TABLE 1

| CHIRON UPDATE | GENOMIC REGION | SAMPLE A00642 DILUTION | RESULT | SAMPLE #401 DILUTION | RESULT | SAMPLE #423 DILUTION | RESULT |
|---|---|---|---|---|---|---|---|
| 1694-1735 | C-100 | 1:800 | POS. | 1:40 | POS. | 1:20 | POS. |
| 1866-1930 | C-100 | 1:100 | POS. | - | - | - | - |
| 1689-1805 | C-100 | 1:500 | POS. | 1:40 | POS. | 1:20 | POS. |
| 1684-1750 | C-100 | 1:500 | POS. | 1:40 | POS. | 1:20 | POS. |
| 1899-1930 | C-100 | 1:50 | POS. | 1:40 | POS. | 1:20 | NEG. |
| 1192-1240 | 33C | 1:800 | POS. | 1:40 | POS. | 1:20 | POS. |
| 1223-1240 | 33C | 1:200 | POS. | 1:40 | POS. | 1:20 | POS. |
| 1-75 | Putative Core | 1:100 | POS. | 1:40 | POS. | 1:20 | POS. |
| 35-75 | Putative Core | 1:50 | NEG. | 1:40 | NEG. | 1:20 | POS. |
| 99-126 | Putative Core | 1:50 | NEG. | 1:40 | NEG. | 1:20 | POS. |
| 1569-1593 | C-100 | 1:25 | NEG. | 1:40 | NEG. | 1:20 | NEG. |
| 1357-1407 | 33C | 1:25 | NEG. | 1:40 | NEG. | 1:20 | NEG. |
| 1418-1457 | 33C | 1:25 | NEG. | 1:40 | NEG. | 1:20 | NEG. |
| 195-262 | Putative Core/ Envelope | 1:50 | NEG. | 1:40 | NEG. | 1:20 | NEG. |
| 230-262 | Putative Core/ Envelope | 1:25 | NEG. | 1:40 | NEG. | 1:20 | NEG. |

A sample is considered positive if the absorbance at 492 nm $\geq$ 4X absorbance value of the negative control (S/N $\geq$ 4.0).

A00642    Human plasma sample convalescent from NANB (HCV) Hepatitis. Patient was clinically diagnosed with NANB and was negative for HBV and HAV markers.

#401    Human paid plasma donor positive by screening assays based on C100-3. No known clinical history.

#423    Human paid plasma donor positive by screening assays based on C100-3. No known clinical history.

There are several known methods using both synthetic and recombinant methodologies to prepare the polypeptides of the present invention which have been found to be immunologically reactive. Preferably, the polypeptides may be prepared using automated synthesizers. The synthesis of p1684 is provided below.

6

## Synthesis of p1684

### H-GRVVLSGKPAIIPDREVLYREFDEMEECSQHLPYIEQGMM-LAEQFKQKALGLLQTASRQAEVIAPAV-OH (see also sequence in Table 3)

The fully protected peptide-resin was assembled on a phenylacetamidomethyl (PAM) resin by stepwise solid phase synthesis (starting with the carboxyl terminal residue) according to the general procedure described by Barany, G. and Merrifield, R.B. in The Peptides, (Gross, E., and Meinhoeffer, T., eds.) 2, 1-284 (1980) Academic Press, New York, NY. The C-terminal amino acid valine (Val) was coupled to the solid support via an oxymethylphenylacetamidomethyl (OMPA) linkage to yield PAM resin which ensures improved stability to prolonged treatment with trifluoroacetic acid (TFA). A BOC-Val-OCH$_2$-Pam-resin (0.78 mmol/g, 0.13 g) was transferred to the reaction vessel of an Applied Biosystems Peptide Synthesizer, model 430A. All subsequent amino acids starting from the carboxyl terminal to N-terminus were coupled in a stepwise manner using Applied Biosystems' small scale rapid cycle protocol. Protected amino acids were coupled using preformed symmetric anhydride chemistry except for asparagine, glutamine, arginine and histidine which were double coupled using N-N'-dicyclohexylcarbodiimide (DCC)/1-hydroxybenzotriazole (HOBT) chemistry. In the first coupling, protected amino acids were coupled using preformed symmetric anhydrides dissolved in dimethylformamide (DMF). The symmetric anhydride of an individual amino acid was formed in methylene chloride followed by solvent exchange to DMF before transferring to the reaction vessel of the peptide synthesizer. The second coupling of symmetric anhydride was also conducted in DMF. The N-amino group of all amino acids used was protected by a t-butyloxycarbonyl (t-BOC) linkage. The side chain functional groups of various amino acids were protected by the following groups:

Arg-Tos (Tosyl)
Lys-2ClZ (2-chlorobenzyloxycarbonyl)
Thr,Ser-Bzl (Benzyl)
Tyr-2BrZ (2-Bromobenzyloxycarbonyl)
Cys-4MeBzl (4-Methylbenzyl)
Asp,Glu-OBzl (0-Benzyl)
His-DNP (Dinitrophenyl)

The fully protected peptide-resin (0.28g) was allowed to swell in methylene chloride (CH$_2$Cl$_2$) for 5 minutes. The peptide-resin was transferred to a manual reaction vessel, treated twice with 5% thiophenol in DMF for twenty minutes each followed by six CH$_2$Cl$_2$ washes for one minute each, and then transferred to the reaction vessel of the synthesizer. The t-BOC protecting group was then removed using 60% TFA/CH$_2$Cl$_2$ according to the manufacturer's protocol and the partially deprotected peptide-resin was then dried overnight under house vacuum at room temperature.

Partially deprotected peptide-resin was then treated with dimethyl sulfide (DMS (1 ml), p-cresol (1 ml), p-thiocresol (0.2 g) and HF (10 mL) at 0°C for one hour to cleave the peptide from the resin support. The HF/DMS and other volatiles were distilled off in vacuo at 0°C. The cleaved peptide and resin were washed three times with 15 ml aliquots of diethyl ether, and the cleaved peptide was extracted by washing three times each with 10 ml aliquots of 40% aqueous acetic acid and 15% aqueous acetic acid, respectively. The aqueous extracts were combined and washed three times with 15 ml aliquots of diethyl ether and then lyophilized to yield a crude peptide.

The crude peptide was analyzed for purity using reversed-phase high performance liquid chromatography on a C$_4$, 4.6 x 30mm column (Brownlee, Applied Biosystems, Inc., Foster City, California), flow rate one ml/minute employing 0.1% aqueous TFA (A) and 100% acetonitrile (B) as the solvent system. The preferred solvent gradient employed for this peptide analysis started with 30% B solvent. The column was maintained at 30% B for one minute followed by an increase over 20 minutes using a linear gradient to 55% B and maintained for one minute. Finally, the column was brought back to 30% B over a two minute period. The presence of peptide in the effluent was monitored simultaneously at 225 nm and 280 nm. The composition of the purified peptide was determined by acid hydrolysis. After removal of the acid, the hydrolysate was analyzed on a Beckmar 6300 amino acid analyzer.

If increased quantities of purified polypeptide were desired, semi-preparative reversed phase high performance liquid chromatography was performed in a similar manner using a C$_4$, 10 x 100 mm column (Brownlee, Applied Biosystems Inc., Foster City, California) using the same aqueous 0.1% TFA (A) and 100% acetonitrile (B) solvent system described above. The preferred solvent gradient for a semi-preparative run started with 27% B at 3 ml/minute for two minutes followed by an increase over 20 minutes using a linear gradient to 50% B. The concentration was maintained at 50% B for one minute and then reduced to 27% B within one minute.

Other peptides described herein were assembled on solid support in a manner analogous to the synthesis described above. The amino acids tryptophan and methionine, if present, were used without any side chain protection. Usually, after incorporating mechionine during the chain assembly, ethanedithiol (0.1% v/v) was added to TFA for all subsequent removal of t-BOC groups. However, if histidine protected by DNP was present in the sequence, ethanedithiol was not added to TFA; instead, indole (1% w/v) was used. Also, after incorporating tryptophan, indole (1% w/v) was added to the TFA solution.

HF cleavage from the resin and purification of the peptides were achieved essentially as described above.

The peptides synthesized as described above were evaluated for their antigenic/immunogenic properties. A summary of the amino acid sequences, beginning with the amino terminus and ending with the carboxy terminus, of immunologically reactive peptides is presented in Table 2.

It is to be noted that polypeptide p1689 is not used alone or in combination with only C100-3 in an assay of the present invention.

## TABLE 2

(Note: H signifies the amino terminus;
OH signifies the carboxyl terminus.)

p1
(1-75)

H-M-S-T-N-P-K-P-Q-K-K-N-K-R-N-T-
N-R-R-P-Q-D-V-K-F-P-G-G-G-Q-I-
V-G-G-V-Y-L-L-P-R-R-G-P-R-L-
G-V-R-A-T-R-K-T-S-E-R-S-Q-
P-R-G-R-R-Q-P-I-P-K-A-R-R-P-
E-G-R-T-OH

alternatively represented by:

H-Met-Ser-Thr-Asn-Pro-Lys-Pro-Gln-Lys-Lys-
Asn-Lys-Arg-Asn-Thr-Asn-Arg-Arg-Pro-Gln-
Asp-Val-Lys-Phe-Pro-Gly-Gly-Gly-Gln-Ile-
Val-Gly-Gly-Val-Tyr-Leu-Leu-Pro-Arg-Arg-
Gly-Pro-Arg-Leu-Gly-Val-Arg-Ala-Thr-Arg-
Lys-Thr-Ser-Glu-Arg-Ser-Gln-Pro-Arg-Gly-
Arg-Arg-Gln-Pro-Ile-Pro-Lys-Ala-Arg-Arg-
Pro-Glu-Gly-Arg-Thr-OH

p35
(35-75)

H-Y-L-L-P-R-R-G-P-R-L-G-V-R-A-T-R-K-T-
S-E-R-S-Q-P-R-G-R-R-Q-P-I-P-K-A-R-R-
P-E-G-R-T-OH

alternatively represented by:

H-Tyr-Leu-Leu-Pro-Arg-Arg-Gly-Pro-Arg-Leu-
Gly-Val-Arg-Ala-Thr-Arg-Lys-Thr-Ser-Glu-
Arg-Ser-Gln-Pro-Arg-Gly-Arg-Arg-Gln-Pro-
Ile-Pro-Lys-Ala-Arg-Arg-Pro-Glu-Gly-Arg-
Thr-OH

## TABLE 2 (CONT'D)

p99
(99-126)

H-S-P-R-G-S-R-P-S-W-G-P-T-D-P-R-R-R-S-
R-N-L-G-K-V-I-D-T-L-OH

alternatively represented by:

H-Ser-Pro-Arg-Gly-Ser-Arg-Pro-Ser-Trp-Gly-
Pro-Thr-Asp-Pro-Arg-Arg-Arg-Ser-Arg-Asn-
Leu-Gly-Lys-Val-Ile-Asp-Thr-Leu-OH

p195
(195-262)

H-R-N-S-T-G-L-Y-H-V-T-N-D-C-P-N-S-S-I-V-Y-
E-A-A-D-A-I-L-H-T-P-G-C-V-P-C-V-R-E-G-N-A-
S-R-C-W-V-A-M-T-P-T-V-A-T-R-D-G-K-L-P-A-T-
Q-L-R-R-H-I-OH

alternatively represented by:

H-Arg-Asn-Ser-Thr-Gly-Leu-Tyr-His-Val-Thr-
Asn-Asp-Cys-Pro-Asn-Ser-Ser-Ile-Val-Tyr-
Glu-Ala-Ala-Asp-Ala-Ile-Leu-His-Thr-Pro-
Gly-Cys-Val-Pro-Cys-Val-Arg-Glu-Gly-Asn-
Ala-Ser-Arg-Cys-Trp-Val-Ala-Met-Thr-Pro-
Thr-Val-Ala-Thr-Arg-Asp-Gly-Lys-Leu-Pro-
Ala-Thr-Gln-Leu-Arg-Arg-His-Ile-OH

p230
(230-262)

H-V-R-E-G-N-A-S-R-C-W-V-A-M-T-P-T-V-A-T-
R-D-G-K-L-P-A-T-Q-L-R-R-H-I-OH

alternatively represented by:

H-Val-Arg-Glu-Gly-Asn-Ala-Ser-Arg-Cys-Trp-
Val-Ala-Met-Thr-Pro-Thr-Val-Ala-Thr-Arg-
Asp-Gly-Lys-Leu-Pro-Ala-Thr-Gln-Leu-Arg-
Arg-His-Ile-OH

### TABLE 2 (CONT'D)

p1192          H-A-V-D-F-I-P-V-E-N-L-E-T-T-M-R-S-P-V-
(1192-1240)    F-T-D-N-S-S-P-P-V-V-P-Q-S-F-Q-V-A-H-
               L-H-A-P-T-G-S-G-K-S-T-K-V-OH

alternatively represented by:

       H-Ala-Val-Asp-Phe-Ile-Pro-Val-Glu-Asn-Leu-
          Glu-Thr-Thr-Met-Arg-Ser-Pro-Val-Phe-Thr-
          Asp-Asn-Ser-Ser-Pro-Pro-Val-Val-Pro-Gln-
          Ser-Phe-Gln-Val-Ala-His-Leu-His-Ala-Pro-
          Thr-Gly-Ser-Gly-Lys-Ser-Thr-Lys-Val-OH

p1223          H-F-Q-V-A-H-L-H-A-P-T-G-S-G-K-S-T-K-V-OH
(1223-1240)

alternatively represented by:

       H-Phe-Gln-Val-Ala-His-Leu-His-Ala-Pro-Thr-
          Gly-Ser-Gly-Lys-Ser-Thr-Lys-Val-OH

p1357          H-Y-V-P-H-P-N-I-E-E-V-A-L-S-T-T-G-E-I-P-F-
(1357-1407)    Y-G-K-A-I-P-L-E-V-I-K-G-G-R-H-L-I-F-C-
               H-S-K-K-K-C-D-E-L-A-A-K-L-OH

alternatively represented by:

       H-Tyr-Val-Pro-His-Pro-Asn-Ile-Glu-Glu-Val-
          Ala-Leu-Ser-Thr-Thr-Gly-Glu-Ile-Pro-Phe-
          Tyr-Gly-Lys-Ala-Ile-Pro-Leu-Glu-Val-Ile-
          Lys-Gly-Gly-Arg-His-Leu-Ile-Phe-Cys-His-
          Ser-Lys-Lys-Lys-Cys-Asp-Glu-Leu-Ala-Ala-
          Lys-Leu-OH

## TABLE 2 (CONT'D)

p1418        H-R-G-L-D-V-S-V-I-P-T-S-G-D-V-V-V-
(1418-1457)   V-A-T-D-A-L-M-T-G-Y-T-G-D-F-D-S-V-
               I-D-C-N-T-C-OH

alternatively represented by:

H-Arg-Gly-Leu-Asp-Val-Ser-Val-Ile-Phe-Thr-
Ser-Gly-Asp-Val-Val-Val-Ala-Thr-Asp-
Ala-Leu-Met-Thr-Gly-Tyr-Thr-Gly-Asp-Phe-
Asp-Ser-Val-Ile-Asp-Cys-Asn-Thr-Cys-OH

p1569        H-D-A-H-F-L-S-Q-T-K-Q-S-G-E-N-L-P-Y-L-V-
(1569-1593)   A-Y-Q-A-T-V-OH

alternatively represented by:

H-Asp-Ala-His-Phe-Leu-Ser-Gln-Thr-Lys-Gln-
Ser-Gly-Glu-Asn-Leu-Pro-Tyr-Leu-Val-Ala-
Tyr-Gln-Ala-Thr-Val-OH

p1684        H-G-R-V-V-L-S-G-K-P-A-I-I-P-D-R-E-V-L-Y
(1684-1750)   R-E-F-D-E-M-E-E-C-S-Q-H-L-P-Y-I-E-Q-G-M
               M-L-A-E-Q-F-K-Q-K-A-L-G-L-L-Q-T-A-S-R-
               Q-A-E-V-I-A-P-A-V-OH

alternatively represented by:

H-Gly-Arg-Val-Val-Leu-Ser-Gly-Lys-Pro-Ala-
Ile-Ile-Pro-Asp-Arg-Glu-Val-Leu-Tyr-Arg-
Glu-Phe-Asp-Glu-Met-Glu-Glu-Cys-Ser-Gln-
His-Leu-Pro-Tyr-Ile-Glu-Gln-Gly-Met-Met-
Leu-Ala-Glu-Gln-Phe-Lys-Gln-Lys-Ala-Leu-
Gly-Leu-Leu-Gln-Thr-Ala-Ser-Arg-Gln-Ala-
Glu-Val-Ile-Ala-Pro-Ala-Val-OH

## TABLE 2 (CONT'D)

p1689
(1689-1805)

H-S-G-K-P-A-I-I-P-D-R-E-V-L-Y-R-E-F
D-E-M-E-E-C-S-Q-H-L-P-Y-I-E-Q-G-M-
M-L-A-E-Q-F-K-Q-K-A-L-G-L-L-Q-T-
A-S-R-Q-A-E-V-I-A-P-A-V-Q-T-N-W-
Q-K-L-E-T-F-W-A-K-H-M-W-N-F-I-S-
G-I-Q-Y-L-A-G-L-S-T-L-P-G-N-P-A-
I-A-S-L-M-A-F-T-A-A-V-T-S-P-L-T-T-S-Q-OH

alternatively represented by:

H-Ser-Gly-Lys-Pro-Ala-Ile-Ile-Pro-Asp-Arg-
Glu-Val-Leu-Tyr-Arg-Glu-Phe-Asp-Glu-Met-
Glu-Glu-Cys-Ser-Gln-His-Leu-Pro-Tyr-Ile-
Glu-Gln-Gly-Met-Met-Leu-Ala-Glu-Gln-Phe-
Lys-Gln-Lys-Ala-Leu-Gly-Leu-Leu-Gln-Thr-
Ala-Ser-Arg-Gln-Ala-Glu-Val-Ile-Ala-Pro-
Ala-Val-Gln-Thr-Asn-Trp-Gln-Lys-Leu-Glu-
Thr-Phe-Trp-Ala-Lys-His-Met-Trp-Asn-Phe-
Ile-Ser-Gly-Ile-Gln-Tyr-Leu-Ala-Gly-Leu-
Ser-Thr-Leu-Pro-Gly-Asn-Pro-Ala-Ile-Ala-
Ser-Leu-Met-Ala-Phe-Thr-Ala-Ala-Val-Thr-
Ser-Pro-Leu-Thr-Thr-Ser-Gln-OH

p1694
(1694-1735)

H-I-I-P-D-R-E-V-L-Y-R-E-F-D-E-
M-E-E-C-S-Q-H-L-P-Y-I-E-Q-G-
M-M-L-A-E-Q-F-K-Q-K-A-L-G-L-OH

alternatively represented by:

H-Ile-Ile-Pro-Asp-Arg-Glu-Val-Leu-Tyr-Arg-
Glu-Phe-Asp-Glu-Met-Glu-Glu-Cys-Ser-Gln-
His-Leu-Pro-Tyr-Ile-Glu-Gln-Gly-Met-Met-
Leu-Ala-Glu-Gln-Phe-Lys-Gln-Lys-Ala-Leu-
Gly-Leu-OH

## TABLE 2 (CONT'D)

p1866     H-F-K-I-M-S-G-E-V-P-S-T-E-D-L-V-N-
(1866-1930)   L-L-P-A-I-L-S-P-G-A-L-V-V-G-V-V-
C-A-A-I-L-R-R-H-V-G-P-G-E-G-A-V-
Q-W-M-N-R-L-I-A-F-A-S-R-G-N-H-V-S-OH

alternatively represented by:

H-Phe-Lys-Ile-Met-Ser-Gly-Glu-Val-Pro-Ser-
Thr-Glu-Asp-Leu-Val-Asn-Leu-Leu-Pro-Ala-
Ile-Leu-Ser-Pro-Gly-Ala-Leu-Val-Val-Gly-
Val-Val-Cys-Ala-Ala-Ile-Leu-Arg-Arg-His-
Val-Gly-Pro-Gly-Glu-Gly-Ala-Val-Gln-Trp-
Met-Asn-Arg-Leu-Ile-Ala-Phe-Ala-Ser-Arg-
Gly-Asn-His-Val-Ser-OH

p1899     H-A-A-I-L-R-R-H-V-G-P-G-E-G-A-V-
(1899-1930)   Q-W-M-N-R-L-I-A-F-A-S-R-G-N-H-V-
S-OH

alternatively represented by:

H-Ala-Ala-Ile-Leu-Arg-Arg-His-Val-Gly-Pro-
Gly-Glu-Gly-Ala-Val-Gln-Trp-Met-Asn-Arg-
Leu-Ile-Ala-Phe-Ala-Ser-Arg-Gly-Asn-His-
Val-Ser-OH

14

## TABLE 2 (CONT·D)

The two underlined Tyr residues are not part of the HCV sequence but are engineered there for ease of iodinating the peptide at a later time).

p380
(380-436)   H   -Gly-Val-Asp-Ala-Glu-Thr-His-Val-Thr-Gly-Gly-Ser-Ala-Gly-His-
            Thr-Val-Ser-Gly-Phe-Val-Ser-Leu-Leu-Ala-Pro-Gly-Ala-Lys-
            Gln-Asn-Val-Gln-Leu-Ile-Asn-Thr-Asn-Gly-Ser-Trp-His-Leu-
            Asn-Ser-Thr-Ala-Leu-Asn-Cys-Asn-Asp-Ser-Leu-Asn-Thr-Gly- OH

p380.LG
(380-436.LG) H   -Gly-Val-Asp-Ala-Glu-Thr-His-Val-Thr-Gly-Gly-Ser-Ala-
            Gly-His-Thr-Val-Ser-Gly-Phe-Val-Ser-Leu-Leu-Ala-Pro-
            Gly-Ala-Lys-Gln-Asn-Val-Gln-Leu-Ile-Asn-Thr-Asn-Gly-
            Ser-Trp-His-Leu-Asn-Ser-Thr-Ala-Leu-Asn-Cys-Asn-Asp-
            Ser-Leu-Asn-Thr-Gly- OH

p447
(447-483)   H.   -Phe-Asn-Ser-Ser-Gly-Cys-Pro-Glu-Arg-Leu-Ala-Ser-Cys-Arg-
            Pro-Leu-Thr-Asp-Phe-Asp-Gln-Gly-Trp-GlyPro-Ile-Ser-Tyr-Ala
            Asn-Gly-Ser-Gly-Pro-Asp-Gln-Arg- OH

p607
(607-627)   H   -Cys-Leu-Val-Asp-Tyr-Pro-Tyr-Arg-Leu-Trp-His-Tyr-Pro-Cys-Thr-
            Ile-Asn-Tyr-Thr-Ile-Phe- OH

p643a
(643-663)   H   -Ala-Cys-Asn-Trp-Thr-Arg-Gly-Glu-Arg-Cys-Asp-Leu-Glu-Asp-
            Arg-Asp-Arg-Ser-Glu-Leu-Ser-_Tyr_-OH

p643b
(643-683)   H   -Ala-Cys-Asn-Trp-Thr-Arg-Gly-Glu-Arg-Cys-Asp-Leu-Glu-Asp-
            Arg-Asp-Arg-Ser-Glu-Leu-Ser-Pro-Leu-Leu-Leu-Thr-Thr-Thr-Gln-
            Trp-Gln-Val-Leu-Pro-Cys-Ser-Phe-Thr-Thr-Leu-Pro- OH

p666
(666-683)   H   -Leu-Leu-Thr-Thr-Thr-Gln-Trp-Gln-Val-Leu-Pro-Cys-Ser-Phe-
            Thr-Thr-Leu-Pro-_Tyr_-OH

p691
(691-714)   H   -His-Leu-His-Gln-Asn-Ile-Val-Asp-Val-Gln-Tyr-Leu-Tyr-Gly-Val-
            Gly-Ser-Ser-Ile-Ala-Ser-Trp-Ala-Ile- OH

p2302       H   -Lys-Lys-Pro-Asp-Tyr-Gln-Pro-Pro-Val-Val-His-Gly-Cys-
(2302-2352)     Pro-Leu-Pro-Pro-Pro-Lys-Ser-Pro-Pro-Val-Pro-Pro-Pro-Lys-
            Lys-Lys-Arg-Thr-Val-Val-Leu-Thr-Glu-Ser-Thr-Leu-Ser-Thr-
            Ala-Leu-Ala-Glu-Leu-Ala-Thr-Arg-Ser-Phe- OH

The polypeptides illustrated in Table 2 may also be prepared in a stepwise fashion or in a fragment coupling protocol using various side chain protection methodologies known to those skilled in the art. The polypeptides may also be prepared using enzymatic methodology.

Further, the polypeptides useful in the practice of this invention may be prepared using recombinant technologies. Briefly, DNA sequences which encode the desired polypeptides are preferably assembled from fragments of the total desired sequence. The fragments are generally prepared using well known automated processes and apparatus. After the complete sequence has been prepared the desired sequence is incorporated into an expression vector which is transformed into a host cell. The DNA sequence is then expressed by the host cell to give the desired polypeptide which is harvested from the host cell or from the medium in which the host cell is cultured. In most cases, the manufactured DNA sequence is assembled using codons which are known to be best expressed in the host cell. When smaller peptides are to be made using recombinant technologies it may be advantageous to prepare a single DNA sequence which encodes several copies of the desired polypeptide in a connected chain. The long chain is then isolated and the chain is cleaved into the shorter, desired sequences.

The amino acid sequence for p1684 is reverse translated to give the codons listed in Table 3 which are optimized (where not inconsistent with assembly and synthesis of fragments) to facilitate high level expression in E. coli. Individual oligonucleotides are synthesized on Applied Biosystem 380A DNA synthesizer using methods and reagents recommended by the manufacturer. These purified oligonucleotides are annealed and ligated together to assemble the entire DNA sequence for digestion with BamH1 and Sal1, allowing ligation into pUC18. The resulting plasmid is suitably transformed into E. coli JM103 cells. Table 3 also lists preferred codons to express p1 and p1223.

TABLE 3

p1

```
                  5                    10                   15                   20
Met Ser Thr Asn Pro Lys Pro Gln Lys Lys Asn Lys Arg Asn Thr Asn Arg Arg Pro Gln
ATG TCT ACC AAC CCG AAA CCG CAG AAA AAA AAC AAA CGT AAC ACC AAC CGT CGT CCG CAG

                  25                   30                   35                   40
Asp Val Lys Phe Pro Gly Gly Gly Gln Ile Val Gly Gly Val Tyr Leu Leu Pro Arg Arg
GAC GTT AAA TTC CCG GGT GGT GGT CAG ATC GTT GGT GGT GTT TAC CTG CTG CCG CGT CGT

                  45                   50                   55                   60
Gly Pro Arg Leu Gly Val Arg Ala Thr Arg Lys Thr Ser Glu Arg Ser Gln Pro Arg Gly
GGT CCG CGT CTG GGT GTT CGT GCT ACC CGT AAA ACC TCT GAA CGT TCT CAG CCG CGT GGT

                  65                   70                   75
Arg Arg Gln Pro Ile Pro Lys Ala Arg Arg Pro Glu Gly Arg Thr
CGT CGT CAG CCG ATC CCG AAA GCT CGT CGT CCG GAA GGT CGT ACC
```

p1223

```
                  5                    10                   15
Phe Gln Val Ala His Leu His Ala Pro Thr Gly Ser Gly Lys Ser Thr Lys Val
TTC CAG GTT GCT CAC CTG CAC GCT CCG ACC GGT TCT GGT AAA TCT ACC AAA GTT
```

p1684

```
                  5                    10                   15                   20
Gly Arg Val Val Leu Ser Gly Lys Pro Ala Ile Ile Pro Asp Arg Glu Val Leu Tyr Arg
GGT CGT GTT GTT CTG TCT GGT AAA CCG GCT ATC ATC CCG GAC CGT GAA GTT CTG TAC CGT

                  25                   30                   35                   40
Glu Phe Asp Glu Met Glu Glu Cys Ser Gln His Leu Pro Tyr Ile Glu Gln Gly Met Met
GAA TTC GAC GAA ATG GAA GAA TGC TCT CAG CAC CTG CCG TAC ATC GAA CAG GGT ATG ATG

                  45                   50                   55                   60
Leu Ala Glu Gln Phe Lys Gln Lys Ala Leu Gly Leu Leu Gln Thr Ala Ser Arg Gln Ala
CTG GCT GAA CAG TTC AAA CAG AAA GCT CTG GGT CTG CTG CAG ACC GCT TCT CGT CAG GCT

                  65
Glu Val Ile Ala Pro Ala Val
GAA GTT ATC GCT CCG GCT GTT
```

16

In order to establish that a alone expresses the DNA sequence, it in grown at 37 °C in 50 ml Luria Broch, in a 250 ml Erlenmeyer flask. When the culture reaches an OD600 of 0.3-0.5, IPTG is added to a final concentration of 1 mM to induce expression. Samples (1.5 ml) are removed at one hour intervals, and the cells are pelleted and resuspended to an OD600 of 10.0 in 2X SDS/PAGE loading buffer. Aliquots (15 ul) of the prepared samples are loaded on a 15% SDS/PAGE gel, the expressed polypeptides separated, and then electrophoretically transferred to nitrocellulose for immunoblotting. The nitrocellulose sheet containing the transferred proteins is incubated with a blocking solution for one hour and incubated overnight at 4 °C with HCV patients' sera diluted in TBS containing 5% E. coli JM103 lysate. The nitrocellulose sheet is washed three times in TBS, then incubated with HRPO-labeled goat anti-human IgG, diluted in TBS containing 10% fetal calf sera. The nitrocellulose is washed three times with TBS and the color is developed in TBS containing 2 mg/ml 4-chloro-1-napthol, 0.02% hydrogen peroxide and 17% methanol. Strong immunoreactive band formation with HCV patients' sera indicates that the synthetic polypeptide in expressed in E. coli in immunologically reactive form.

Preferred formats for assays using the polypeptides described above are provided in the following examples. Example 1 describes a confirmatory assay. Example 2 describes a combination assay. Example 3 describes a synthetic polypeptide-based assay. Example 4 describes an immunodot assay. Example 5 describes a competition assay. Example 6 describes an EIA assay in which peptides 380-436 and 447-483, 643-683 and 2302-2352 are used. Example 7 describes an EIA utilizing peptide p380.LG. Example 8 describes an EIA utilizing peptide 2302 (NS-5) compared to an EIA utilizing antigens NS3 (CKS-33C), NS4 (C-100) or CORE (CRS-CORE). Example 9 describes the PEPSCAN protocol followed.

## Example 1. CONFIRMATORY ASSAY

The confirmatory assay uses at least two polypeptides containing HCV antigenic epitopes which are preferably prepared and isolated from different sources. One polypeptide is used to screen serum or plasma samples. The other polypeptide is used to confirm the presence of a HCV antibody in a sample initially identified as containing a HCV antibody by the screening procedure.

In the presently preferred confirmatory assay, the screening procedure uses a recombinant C100-3 polypeptide. The C100-3 recombinant polypeptide is believed to contain multiple epitopes as well as an immunodominant region defined by the 1689-1806 amino acid sequence. The C100-3 polypeptide is expressed in recombinant yeast cells and isolated from the cell extract as described in EPA publication Number 0 318 216. Other recombinant polypeptides containing amino acid sequences essentially duplicative of C100-3 may also be used.

The other peptide used in the confirmatory assay is a synthetic peptide selected from the group consisting of p1, p35, p99, p1192, p1223, p1684, p1689, p1694, p1866 and p1899, although p1689 cannot be used alone with C100-3 as part of the present invention. Preferably the peptide is p1684 or p1866. These peptides were prepared following procedures described above. In the confirmatory assay, both C100-3 and the synthetic peptides, p1684, p1694 or p1866, were separately coated onto polystyrene beads. A combination of synthetic peptides coated on a polystyrene bead may also be used if desired.

The polystyrene beads are first washed with distilled water and propanol then incubated with crude or purified HCV synthetic peptides diluted to 0.1-20.0 ug/ml in a 0.1 M solution of an appropriate buffer containing about 0.4-0.5 M NaCl, about 0.0022% Triton® X-100 and adjusted to about pH 6.5-10.0. The following buffers, tris, $NaH_2PO_4 \cdot H_2O$, boric acid, and citrate buffers are preferred and are optimized for each peptide; preferred buffers, pH and coating concentration for the synthetic peptides are listed in Table 4. Successful coatings have also been accomplished with lower or higher pH. The beads are incubated in the antigen solution for about two hours at 38-42 °C, washed in phosphate buffer solution (PBS) and soaked in 0.1% Triton® X-100 in PBS for sixty minutes at 38-42 °C. The beads are then washed two times in PBS, overcoated with a solution of 5% (w/v) bovine serum albumin in PBS for sixty minutes and washed three times with PBS. Finally, the beads are overcoated with 5% (w/v) sucrose in PBS and dried under nitrogen or air.

The peptides are each individually coated onto polystyrene beads and used in an antibody capture format. Ten microliters of sample are added to the wells of a reaction tray along with 400 $\mu$l of a sample diluent and a peptide coated bead. The sample diluent consists of about 10% (v/v), or less, bovine serum and about 20% (v/v), or less, goat serum in 20 mM Tris phosphate buffer containing 0.20%, or less, (v/v) Triton® X-100, 3% (w/v), or less, bovine serum albumin. When the recombinant yeast C100-3 polypeptide is used, antibodies to yeast antigens which may be present in a sample are reacted with yeast extracts which are added to the sample diluent (typically about 200 $\mu$g/ml). The addition of yeast extracts to the sample diluent is used to prevent false positive results. The final material is sterile filtered and filled in plastic bottles, and preserved with 0.1% sodium azide.

After one hour of incubation at 40 °C, the beads are washed and 200 µl of conjugate is added to the wells of the reaction tray.

TABLE 4

| PEPTIDE | COATING CONCEN. ug/ml | COATING BUFFER | OTHER COMPONENTS IN COATING SOLUTION |
|---|---|---|---|
| 1-75 | 2.0 | 0.1M NaPHOSPHATE pH 6.5 | 0.4M NaCl, .0022%TRITON® X-100 |
| 35-75 | 2.0 | 0.1M NaPHOSPHATE pH 6.5 | 0.4M NaCl, .0022%TRITON® X-100 |
| 99-126 | 2.0 | 0.1M NaPHOSPHATE pH 6.5 | 0.4M NaCl, .0022%TRITON® X-100 |
| 195-262 | 2.0 | 0.1M NaPHOSPHATE pH 6.5 | 0.4M NaCl, .0022%TRITON® X-100 |
| 230-262 | 2.0 | 0.1M NaPHOSPHATE pH 6.5 | 0.4M NaCl, .0022%TRITON® X-100 |
| 1357-1407 | 2.0 | 0.1M BORIC ACID pH 9.0 | 0.4M NaCl, .0022%TRITON® X-100 |
| 1418-1457 | 2.0 | 0.1M BORIC ACID pH 9.0 | 0.4M NaCl, .0022%TRITON® X-100 |
| 1569-1593 | 3.0 | 0.1M TRIS/HCl pH 8.5 | 0.5M NaCl, .0022%TRITON® X-100 |
| 1899-1930 | 2.0 | 0.1M TRIS/HCl pH 8.5 | 0.5M NaCl, .0022%TRITON® X-100 |
| 1192-1240 | 2.0 | 0.1M BORIC ACID pH 9.0 | 0.4M NaCl, .0022%TRITON® X-100 |

## TABLE 4 (CONT'D)

| | | | |
|---|---|---|---|
| 0.1M BORIC ACID pH 9.0 | 0.4M NaCl, .0022% TRITON® X-100 | 5.0 | 1223-1240 |
| 0.1M BORIC ACID pH 10.0 | 0.4M NaCl, .0022% TRITON® X-100 | 1.0 | 1684-1750 |
| 0.1M BORIC ACID pH 10.0 | 0.4M NaCl, .0022% TRITON® X-100 | 1.0 | 1689-1005 |
| 0.1M TRIS/HCL pH 8.5 | 0.5M NaCl, .0022% TRITON® X-100 | 3.0 | 1694-1735 |
| 0.1M TRIS/HCl pH 8.5 | 0.5M NaCl, .0022% TRITON® X-100 | 0.75 | 1866-1930 |
| 0.1 M TRIS/HCl pH 8.5 | 0.9% NaCl | 3.0 | 380-436 |
| | | 3.0 | 447-483 |
| 0.1 M TRIS/HCl pH 8.5 | 0.5 M NaCl 0.0022% TRITON®X-100 | 3.0 | 643-683 |
| 0.1 M Borate | 0.4 M NaCl 0.0022% TRITON®X-100 | 3.0 | 2302-2352 |

The preferred conjugate is goat anti-human IgG horseradish peroxidase conjugate. Concentrated conjugate is purchased from Kirkegaard and Perry Laboratories, Inc., Gaithersburg, Maryland, and is titered to determine a working concentration. A twenty-fold concentrate of the working conjugate solution is then prepared by diluting the concentrate in diluent. The conjugate diluent includes 10% (v/v) bovine serum, 10% (v/v) goat serum and 0.15% Triton®-X100 in 20 mM Tris buffer, pH 7.5 with 0.01% gentamicin sulfate, pink dye and antifungal agents as preservatives. The conjugate is sterile filtered and filled in plastic bottles.

After one hour of incubation with the conjugate at 40 °C, the beads are washed, exposed to the OPD substrate for thirty minutes at room temperature and the reaction terminated by the addition of 1 N $H_2SO_4$. The absorbance is read at 492 nm.

Samples found to be repeatably reactive by a screening assay using the polypeptide C100-3 are tested in duplicate using p1684 or p1689 coated beads. Reactive specimens are considered confirmed samples. Samples not reacting with p1684 or p1689 are tested in duplicate with p1694 and p1866 beads. Samples reacting with one or both of these peptides are considered confirmed: Those specimens not reacting with any of these peptides are considered nonconfirmed.

In order to maintain acceptable specificity, the cutoff for the assay should be at least 5-15 standard deviations above the absorbance value of a normal population mean. Consistent with these criteria, a cutoff for the assay may be selected which clearly separated most of the presumed "true negatives" from "true positive" specimens. A general cutoff value may be calculated as about 2.1 to 8 times the negative control mean absorbance value.

Confirmatory Assay Performance

1. Intravenous Drug User Samples

Samples were collected from a population of intravenous drug users enrolled in an NIH-funded study. The population consisted of individuals who were acknowledged users of intravenous drugs selected over a two-year period from patients at the Edward Hines Jr. Veteran's Administration Hospital in Maywood, Illinois by Dr. Connie Pachucki and members of the Infectious Diseases staff.

As illustrated in Table 5, a total of 296 specimens, each obtained fron a single donor, were screened using recombinant yeast C100-3 polypeptide. A total of 271 of 296 (91.6%) specimens initially tested positive; upon retesting, 269 of 271 (99.3%) were repeat positives.

Confirmatory testing indicated that 263 of 269 (97.8%) of the repeat positives were reactive with p1689, five specimens were non-reactive with p1689, and one specimen was not tested with any of the confirmatory polypeptides. Four of the five specimens which were non-reactive with p1689 were reactive with p1866 only; one specimen which was non-reactive with p1689 was reactive with p1694 only.

All specimens which were repeatably reactive were confirmed reactive in assays using the HCV synthetic peptides.

TABLE 5

| INTRAVENOUS DRUG USERS SAMPLES | | | | | |
|---|---|---|---|---|---|
| Confirmatory Testing | | | | | No. of Repeat Positives Confirmed |
| C100-3 Initial Positive | C100-3 Repeat Positive | p1689 | p1866 | p1694 | |
| 271/296 | 269/271 | 263/269 | 4/5 | 1/5 | 268/269 |

2. Chimpanzees Samples

Confirmatory assays were used to evaluate 92 samples from six chimpanzees. All were initially reactive with recombinant C100-3. (Duplicate, repeat testing of chimp sera was not done because of the rare nature of these specimens and their utility for serological studies with other HCV antigens). Eighty-three of 92 (90.2%) specimens were confirmed reactive using p1689. Confirmation of initial reactives improved to 96.7% (89 of 92) when repeat testing with p1694 and p1866 was done.

3. Chiron Corporation Non-A, Non-B Hepatitis Virus Proficiency Panel #2

A proficiency panel comprised of neat and diluted human plasma including specimens containing antibodies to HCV C100-3 was provided by scientists at the Chiron Corporation (12 specimens). This panel contains specimens ranging from low to high reactivity in other assays, non-reactive presumed "true negative" specimens, and reactive specimens diluted to give low-level or negative results.

Results using the confirmatory assay on the Chiron Corporation Non-A, Non-B Hepatitis Virus Proficiency Panel #2 indicated 9 of 9 (100%) of the specimens reactive by the preliminary screening assay are confirmed by p1689. All negative specimens were non-reactive.

4. Confirmatory Testing on NANB Panel II

A panel of highly pedigreed human sera from Dr. H. Alter, NIH, Bethesda, MD, containing infectious HCV sera, negative sera and other disease controls were tested. A total of 44 specimens were present in the panel.

All specimens (16/16, 100%) reactive in the assay using C100-3 were confirmed by p1689, as shown in Table 6. Again, there were no nonspecific reactives as all pedigreed negative or "other disease" controls were non-reactive in the peptide assay.

**TABLE 6**

**COMPARISON OF p1689 RESULTS WITH HCV SCREENING
ASSAY RESULTS ON NANB PANEL II (H. ALTER, NIH).**

| SAMPLE | C100-3 | | ORTHO C100-3 | p1689 EIA |
|---|---|---|---|---|
| | MANUAL S/CO | MACHINE S/CO | S/CO | S/CO |
| 1 | >5.88 | >6.47 | >6.38 | >8.33 |
| 2 | 0.63 | 0.93 | 0.27 | 0.45 |
| 3 | >5.88 | >6.47 | >6.38 | >8.33 |
| 4 | >5.88 | >6.47 | >6.38 | >8.33 |
| 5 | 0.43 | 0.35 | 0.18 | 0.43 |
| 6 | >5.88 | >6.47 | >6.38 | >8.33 |
| 7 | 0.46 | 0.73 | 0.36 | 0.32 |
| 8 | 0.41 | 0.50 | 0.32 | 0.38 |
| 9 | 1.87 | 2.21 | 0.91 | 2.84 |
| 10 | 0.35 | 0.41 | 0.32 | 0.30 |
| 11 | 0.48 | 0.45 | 0.27 | 0.46 |
| 12 | 0.32 | 0.41 | 0.17 | 0.39 |
| 13 | 0.48 | 0.69 | 0.32 | 0.51 |
| 14 | 0.37 | 0.40 | 0.19 | 0.32 |
| 15 | >5.88 | >6.47 | >6.38 | >8.33 |
| 16 | >5.88 | >6.47 | >6.38 | >8.33 |
| 17 | 0.34 | 0.40 | 0.20 | 0.44 |
| 18 | 3.01 | 3.68 | 0.68 | 6.80 |
| 19 | 0.74 | 0.81 | 0.53 | 0.72 |
| 20 | 0.53 | 0.59 | 0.28 | 0.33 |
| 21 | >5.88 | >6.47 | >6.38 | >8.33 |
| 22 | 0.24 | 0.26 | 0.20 | 0.23 |
| 23 | >5.88 | >6.47 | >6.38 | >8.33 |
| 24 | 0.69 | 0.64 | 0.53 | 0.70 |
| 25 | 0.50 | 0.60 | 0.49 | 0.40 |
| 26 | 3.41 | 4.11 | 0.77 | 6.61 |
| 27 | 0.62 | 0.74 | 0.30 | 0.65 |
| 28 | 0.61 | 0.77 | 0.08 | 0.47 |
| 29 | 0.34 | 0.42 | 0.13 | 0.33 |
| 30 | 1.58 | 2.40 | 1.26 | 2.65 |
| 31 | 0.32 | 0.35 | 0.22 | 0.37 |
| 32 | >5.88 | >6.47 | >6.38 | >8.33 |
| 33 | 0.45 | 0.48 | 0.24 | 0.45 |
| 34 | >5.88 | >6.47 | >6.38 | >8.33 |
| 35 | >5.88 | >6.47 | >6.38 | >8.33 |
| 36 | 0.37 | 0.36 | 0.21 | 0.40 |
| 37 | 0.40 | 0.46 | 0.24 | 0.52 |
| 38 | >5.88 | >6.47 | >6.38 | >8.33 |
| 39 | 0.40 | 0.49 | 0.80 | 0.46 |
| 40 | 0.53 | 0.50 | 0.30 | 0.56 |
| 41 | 0.41 | 0.28 | 0.18 | 0.32 |
| 42 | 0.52 | 0.56 | 0.38 | 0.60 |
| 43 | 0.28 | 0.30 | 0.38 | 0.33 |
| 44 | 0.44 | 0.57 | 0.38 | 0.83 |

Data presented demonstrate the efficacy of the confirmatory assay for detection of antibodies to HCV antigens. The current assay is both sensitive and specific for detection of antibodies to HCV antigens.

The data further support the utility of the confirmatory strategy using synthetic peptides. The synthetic peptides serve as an independent source of antigen for use in immunoassays. The ability to confirm an average of 99% of repeatably active specimens in high risk or pedigreed positive HCV panels, establishes

22

the utility of this strategy.

Example 2. COMBINATION ASSAY

The combination assay uses more than one polypeptide antigen coated on the same bead. To prepare multiple polypeptide-containing beads, the polystyrene beads described in Example 1 are contacted simultaneously with the polypeptide in appropriate buffer solutions. After the beads have been contacted with the polypeptides, the bead is treated further as described above.

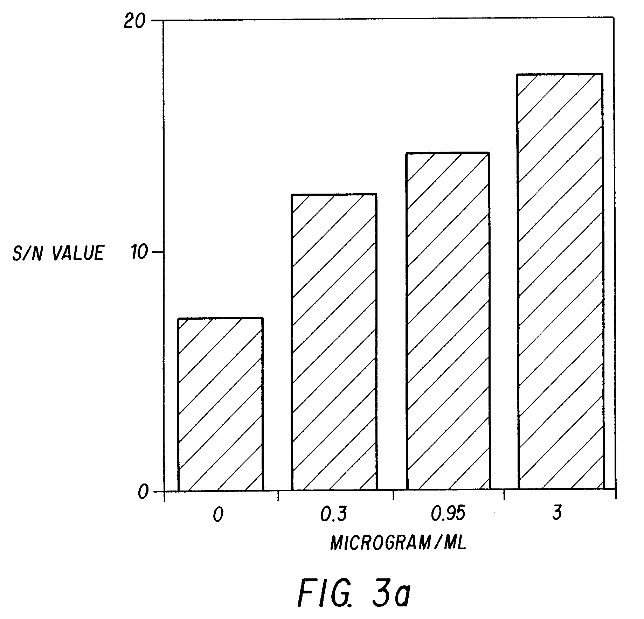

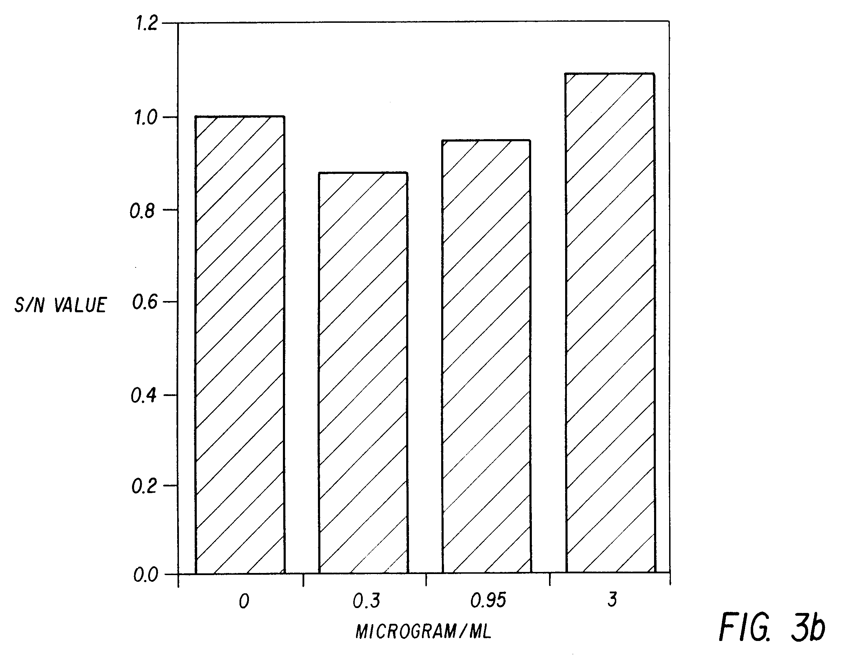

For a polystyrene bead containing both C100-3 and p1694 the sensitivity of the assay increases. As graphically illustrated in Figure 3a, adding about 0.3, 0.95, and 3 micrograms of p1694 to the coating solution, respectively, shows a significant increase in the signal when the detection procedures of Example 1 are utilized. Figure 3b graphically illustrates the data which show no corresponding increase in the signals (such as may attend non-specific binding) generated from negative human plasma.

Example 3. SYNTHETIC POLYPEPTIDE-BASED ASSAY

In the following discussion it is to be noted that assays depending on the p1689 polypeptide are presented for illustrative purposes and are not part of the invention.

The use of synthetic polypeptides which contain epitopes of HCV antigens provide immunological assays which have increased sensitivity and may be more specific than HCV immunological assays using the SOD fusion polypeptide C100-3. The use of shorter amino acid sequences on polystyrene bead provides an increase in sensitivity.

The increased sensitivity of an assay employing synthetic polypeptide compared to recombinant C100-3 polypeptide was demonstrated in a serial dilution study. The serial dilution study employed fifteen samples which were identified as having antibodies to HCV antigens using a recombinant C100-3 screening assay. Each positive sample was assayed using recombinant C100-3 polypeptide in one assay and p1689 polypeptide in a second assay, and the samples were then diluted twofold until the S/CO value was less than one. In twelve samples the p1689 polypeptide gave increased sensitivity (larger S/CO values) at all dilutions. In two samples, the p1689 polypeptide and the recombinant yeast C100-3 polypeptides were essentially equivalent. In one sample, the p1689 polypeptide gave a negative response to a positive sample at all dilutions.

Additional studies on samples from serial bleeds of three chimps which developed an acute resolved case of HCV infections and three chimps which developed chronic HCV infections showed different immunological responses believed to be due to both the type of infection and the polypeptide used in the assay. This study assayed serum from serial bleeds of six chimps inoculated with HCV. The assay protocols were similar to those described in Example 1 above with the following differences.

The antibodies, IgG, IgM and IgA were detected using affinity purified goat antibodies to human IgG, IgM and IgA coupled to horseradish peroxidase (Kirkegaard & Perry Laboratories, Inc., Gaithersburg, Maryland) which were used at working concentrations of 0.2 ug/ml Anti-IgG, 0.5 ug/ml Anti-IgM and 0.2 ug/ml Anti-IgA. Serum dilutions for each assay were 1:41 for IgG, 1:101 for IgM, and 1:41 for IgA.

The polypeptides that were used in the study include C100-3, p1694, p1684, p1689, and p1866.

Briefly, beads containing the polypeptides were incubated with diluted serum for one hour at 40 °C, the beads were washed and incubated with the appropriate goat antibody for one hour at 40°C. The beads were washed again and the assay was developed by incubating the beads with OPD for thirty minutes at room temperature. The color development was quenched with 1 N sulfuric acid and the results read at 492 nm.

All chimps developed antibodies that were detected by C100-3, p1684, and p1866 within 7 to 17 week post-inoculation (WPI). Within each chimp, IgG antibody reacting with C100-3, p1684, and p1866 appeared at approximately the same time. The response to p1694 and p1866 was variable within that time period with indications that antibody to these two peptides can be either undetectable or significantly delayed following HCV infection. These data suggest that, out of the five peptides tested, antibody to C100-3, p1684, or p1689 would be the earliest and most consistent serologic indicator of HCV infection.

IgM antibody was detected in only three of the six chimps studied. The response of each of the three animals to C100-3, p1684, p1689 and p1694 was detected in 7 to 10 WPI whereas IgM antibody to p1866 was undetectable in two chimps and delayed in the third. All IgM responses were short lived with levels falling below positive (S/N less than 3.0) within 2 to 22 weeks.

The explanation and significance of finding IgM antibodies in 3 chimps with acute resolved disease while not detecting IgM antibodies in 3 chimps with chronic infection is unexpected. Preliminary experimental results indicated that false negative IgM results due to preferential IgM binding is an unlikely explanation. If the pattern observed in these 6 chimps with the five peptides holds true, antibody assays will provide important HCV prognostic information.

A positive IgA response (S/N greater than 3.0) was detected in only 2 of the 6 chimps and proved to be either biphasic or significantly later than the IgG or IgM response. Although these 2 chimps had chronic disease no conclusions regarding the significance of IgA antibodies can be made since sera from the three resolved chimps is available only through 30 to 40 WPI.

The results show the polypeptides when used to assay for antibodies to HCV antigens are useful to follow the progression of HCV infection and that the polypeptides exhibit unexpected sensitivity to different antibodies generated during the clinical progression of HCV infection.

Example 4. IMMUNODOT ASSAY.

The immunodot assay of the invention excludes those assays relying only on a combination of C100-3 and p1689 polypeptides.

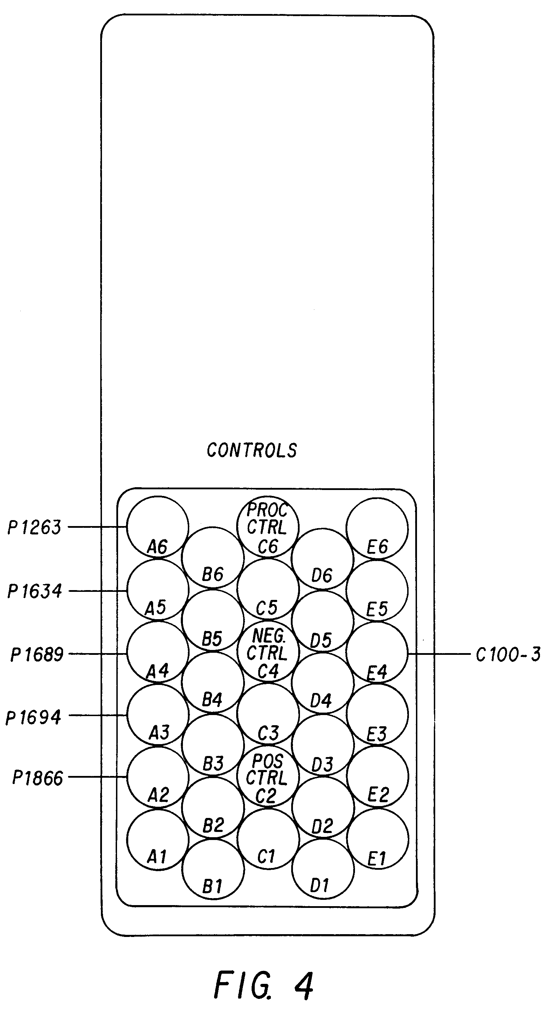

The immunodot assay system uses a panel of purified synthetic polypeptides placed in an array on a nitrocellulose solid support. The prepared solid support is contacted with a sample and captures specific antibodies to HCV antigens. The captured antibodies are detected by a conjugate-specific reaction. Preferably, the conjugate-specific reaction is quantified using a reflectance optics assembly within an instrument which has been described in U.S. Patent Application Serial No. 07/227,408, filed August 2, 1988. The related U.S. Patent Applications Serial Nos. 07/227,272, 07/227,586, and 07/227,590 further describe specific methods and apparatus useful to perform an immunodot assay. Briefly, a nitrocellulose-base test cartridge is treated with multiple antigenic polypeptides. A test cartridge which may be used in an automated process for performing an immunodot assay described above is illustrated in Figure 4. Each polypeptide is contained within a specific reaction zone on the test cartridge. After all the antigenic polypeptides have been placed on the nitrocellulose, excess binding sites on the nitrocellulose are blocked. The test cartridge is then contacted with a sample such that each antigenic polypeptide in each reaction zone will react if the sample contains the appropriate antibody. After reaction, the test cartridge is washed and any antigen-antibody reactions are identified using suitable well known reagents.

As described in the patent applications listed above, the entire process is amenable to automation. The specifications of these applications related to the methods and apparatus for performing an immunodot assay are incorporated by reference herein.

In a preferred immunodot assay, the synthetic polypeptides p1223, p1684, p1689 and p1866 were diluted into an aqueous buffered solution (polypeptide diluent: 0.03% Triton® X-100 and 0.1% sodium azide in 50 mM Hepes buffer, pH 7.6) and applied to a preassembled nitrocellulose test cartridge at about 40 ng in each reaction zone. After drying the cartridge overnight at room temperature, the nonspecific binding capacity of the nitro-cellulose phase was blocked. The blocking solution contained 1% porcine gelatin, 1% casein enzymatic hydrolysate, 5% Tween®-20, 0.1% sodium azide, 0.5 M sodium chloride and 20 mM Tris, pH 7.5.

Test cartridges were incubated with samples 00642 and 423 (see Table 1) and ALT 27. The sample ALT 27 was obtained from a volunteer donor having elevated alanine aminotransferase levels. After sample incubation, sequential incubations with a biotin-conjugated goat anti-human immunoglobulin-specific antibody, an alkaline phosphatase-conjugated rabbit anti-biotin specific antibody, and 5-bromo-4-chloro-3-indolyl phosphate produced a colored product at the site of the reaction.

A detectable reaction is defined by the formation of a visually discernable product at the antigen site on the array; when quantified by the instrument, a reflectance density (Dr) value of greater than or equal to approximately 0.0150 above background is obtained. None of the tested polypeptides elicited a detectable reaction with a negative control serum that was previously demonstrated negative for antibodies to HCV antigens using a recombinant C100-3 polypeptide.

A reaction with each of the synthetic polypeptides p1684, p1689, p1694 and p1866 occurred when the prepared test cells were incubated with either sample 00642 (1:100 dilution in negative serum) or sample 423 (1:40 dilution in negative serum). Polypeptide p1223, in addition to polypeptides p1684, p1689, p1694 and p1866 demonstrated a significant reaction with the elevated ALT 27 specimen. In all specimens, highest reactivity was obtained with p1689. Enhanced reactivity of polypeptide p1684 with sample 00642 was achieved through subtle modification of the antigen dilution (the modified polypeptide diluent was 0.5 M sodium chloride, 0.0022% Triton® X-100 and 0.1 M Tris/HCl, pH 8.5).

The net reflectance (Dr) for a test cartridge containing the polypeptides p1223, p1684, p1689, p1694, and p1866 which indicate a positive or negative response is set out in Table 7.

## TABLE 7

| Sample | +/- Values Based on S/N | | | | |
|---|---|---|---|---|---|
| | p1223 | p1684 | p1689 | p1694 | p1866 |
| Neg Ctrl | - <br> - | - <br> - | - <br> - | - <br> - | - |
| Sample 00642 (1:100) | - <br> - | + <br> + | + <br> + | + <br> + | + <br> + |
| Sample 423 (1:40) | - | + | ++ | + | + |
| Sample ALC27 | + <br> + | ++ <br> ++ | +++ <br> +++ | ++ <br> ++ | ++ <br> ++ |

+/- Values Based on S/N

-     Dr/Bkg < 2.5
+     2.5 < Dr/Bkg < 100
++    100 < Dr/Bkg < 1000
+++   Dr/Bkg > 1000

| Sample | NET REFLECTANCE DENSITY (D) | | | | |
|---|---|---|---|---|---|
| | p1223 | p1684 | p1689 | p1694 | p1866 |
| Neg Ctrl | -.0051 <br> -.004 | -.0042 <br> .0004 | .0047 <br> .0068 | .0012 <br> .0000 | .0134 <br> .0119 |
| Sample 00642 (1:100) | .0011 <br> .0002 | .0281 <br> .0368 | .4401 <br> .4552 | .0748 <br> .0853 | .1444 <br> .1564 |
| Sample 423 (1:40) | .0073 | .1501 | 1.5389 | .1922 | .0325 |
| Sample ALC27 | .3040 <br> .1469 | 10.40 <br> 9.619 | 29.64 <br> 30.20 | 13.00 <br> 13.26 | 7.450 <br> 7.007 |

Example 5. COMPETITION ASSAY.

The synthetic peptides containing antigenic HCV epitopes are useful for competition assays. To perform a neutralization assay, peptides other than p1689 representing epitopes within the C100-3 region such as p1694 or p1684 are solubilized and mixed with a specimen diluent to a final concentration of 0.5-50 $\mu$g/ml.

25

Ten microliters of specimen or diluted specimen is added to a reaction well followed by 400 $\mu$l of the specimen diluent containing peptide and if desired, the mixture may be pre-incubated for about fifteen minutes to two hours. A bead coated with C100-3 antigen of HCV is then added to the reaction well and incubated for one hour at 40 °C. After washing, 200 $\mu$l of a peroxidase labeled goat anti-human IgG in conjugate diluent is added and incubated for one hour at 40 °C. After washing, OPD substrate is added and incubated at room temperature for thirty minutes. The reaction is terminated by the addition of 1 N sulfuric acid and the absorbance read at 492 nm.

Samples containing antibodies to the C100-3 antigen generate a reduced signal caused by the competitive binding of the peptides to these antibodies in solution. The percentage of competitive binding may be calculated by comparing the absorbance value of the sample in the presence of a synthetic peptide to the absorbance value of the sample assayed in the absence of a synthetic peptide at the same dilution.

EXAMPLE 6. EIA ASSAY

Beads were coated with either peptides 380-436 and 447-483, 643-683 and 2302-2353 according to the method described in Example 1, except that peptides 380-436 and 447-483 were coated simultaneously on the same solid phase, both sequences being from the putative envelope region of HCV. Either peptide alone had activity in this type of assay. EIA was performed using each bead configuration described herein. The EIA method performed was as is described in Example 1, with the cutoff set at four times the negative control value. Table 8 presents data obtained from these assays in which serum specimens from patients diagnosed with chronic NANBH were assayed.

TABLE 8

| ANTIGEN # POS./NO. TESTED | | |
|---|---|---|
| p380 | p643b | p2302 |
| 70/165 (42%) | 62/165 (38%) | 102/165 (62%) |

-- EXAMPLE 7 EIA UTILIZING p380.LG AND p380.

Beads were coated either with p380.LG or p380 according to Example 1. An EIA following the procedure of Example 1 was used to assay samples. As can be seen by the data presented in Table 9, the p380.LG peptide detected antigen in specimens that were negative to p380. The p380.LG sequence is highly variable in this region. Therefore, there is reasonable probability that differentiation between HCV "serotypes" based on reactivity of human specimens to one or the other of these envelope region peptide sequences is possible. The data of Table 10 suggest that p380.LG can detect chronically infected HCV patients who are negative to p380.

TABLE 9

| SAMPLE | p380 | | p380LG | |
|---|---|---|---|---|
| | OD | S/N | OD | S/N |
| #8 | .155 | 2.12 | .399 | 5.87 |
| #28 | .246 | 3.37 | .950 | 13.97 |
| #23 | .114 | 1.56 | .458 | 6.74 |

EXAMPLE 8. EIA UTILIZING p2302.

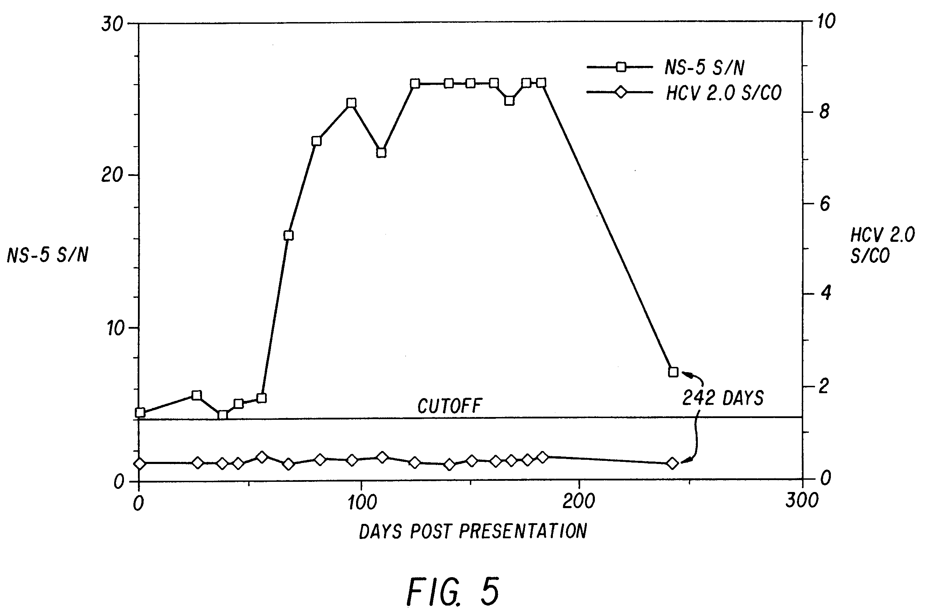

Beads were coated with either p2303 or "HCV2.0S/CO" according to the method of Example 1. The bead coated with "HCV 2.0/SCO" comprised antigen from the NS3 (CKS-33C), NS4 (C-100) and Core (CKS-CORE) regions of the HCV genome. A patient sample which exhibited seroconversion to p2302, but not to HCV.20 S/CO, is shown in Figure 5. Thus, this peptide improves the ability to detect HCV infected individuals.

EXAMPLE 9. PEPSCAN PROTOCOL

NS1 region of HCV genome from a.a. 600-720 was mapped with PEPSCAN analysis, which is serological analyses of series of overlapping peptides spanning the protein sequence to identify immunogenic domains. A total of 106 overlapping hexamer peptides were synthesized on polypropylene pins following the manufacturer's instructions (Cambridge Research Bioscience, Valley Stream, N.Y.). Fab dimers of IgG purified from sera of individuals seropositive for HCV were tested with these peptides. Based on the reactivity in EIA (performed as described by the manufacturer) four peptide sequences were selected as illustrated in Table 10.

Each of these peptides were synthesized by a stepwise solid phase synthesis, starting with the Carboxy terminus residue. A panel of sera positive for antibodies to C-100 protein of CHV was tested for their reactivity to NS1 peptide by microtiter EIA as described below.

EIA PROTOCOL

Wells of microtiter plates were coated with 100 $\mu$l of the peptide at 10 $\mu$g/ml in 0.02M bicarbonate buffer, pH 9.5 at ambient temperatures for 12-16 hrs. After washing with Phosphate buffered saline which also contained 0.01% Sodium Dodecyl sulphate (SDS) and 0.05% Tween-20® (available from BioRod Laboratories, Richmon, CA.), free sites were overcoated with 1% BSA in bicarbonate buffer pH 9.5. Plates were stored at 4 C following a final wash.

Sera from individuals seropositive for antibodies to HCV C-100 were serially diluted in 100 $\mu$l of a buffer containing 20MM sodium phosphate, pH 7.4, 0.15M NaCl, 20% normal goat serum, 10% fetal calf serum, 5 MM EDTA, 10MM EGTA, 50MM Tris, 0.2% Tween®-20 with sodium azide as preservative, pH 6.8. The diluted sera were reacted with peptides in microtiter wells for 3 hours at 37°C or overnight at ambient temperatures. The plates were washed and 100 $\mu$l of appropriately diluted goat anti-mouse (HH) Horseradish Peroxidase (HRPO)-conjugated antibody (Jackson immunochemicals, West Grove, PA) was added. The plates were incubated at 37°C for 2 hours. After a final wash, 100 $\mu$l of O-phenylenediamine 2HCl (OPD) color reagent was added. The reaction was carried out at room temperature in the dark for 20-25 minutes, and stopped by the addition of 100 $\mu$l of IN H SOu. The absorbance of the reaction mixture recorded at 492 NM. A negative control which was previously confirmed to be negative for HCV infection was included with each plate in triplicate. The sample was considered reactive if the absorbance of the sample at a 1:2000 dilution was three times the absorbance of the negative control at the same dilution. Table 11 illustrates the reactivity of these samples with each of the peptides.

The legend for Table 12 is as follows:

+ = Sample showing A492 3 X neg. control

+ + = Sample titering to 1:5000 dilution

+ + + = Strong reactivity with sample titering to 1:10,000 dilution.

It is envisioned that these peptides may be used for the development of unique polyclonal and monoclonal antibodies. Other variations of applications and modifications of the specific embodiments of the invention as set forth herein will be apparent to those skilled in the art. Accordingly, the invention is intended to be limited only in accordance with the appended claims.

TABLE 10

| AMINO ACID SEQUENCE OF PEPTIDES SELECTED FROM THE NS1 REGION OF HCV GENOME (A.A. 600-720) BASED ON PEPSCAN ANALYSIS | |
| --- | --- |
| A.A. NO. OF HCV GENOME | PEPTIDE SEQUENCE* |
| 607-627 | CLVDYPYRLWHYPCTINYTIF |
| 643-663 | ACNWTRGERCDLEDRDRSELSY |
| 666-683 | LLTTTQWQVLPCSFTTLPY |
| 691-714 | HLHQNIVDVQYLYGVGSSIASWAI |

* See also Table 2

TABLE 11

| REACTIVITY OF A PANEL OF HCV SEROPOSITIVE SAMPLES WITH NS1 PEPTIDES BY MICROTITER EIA | | | | |
|---|---|---|---|---|
| SAMPLE ID* | PEPTIDES SELECTED FROM NS1 REGION (A.A. NUMBERS) | | | |
| | 607-627 | 643-663 | 666-683 | 691-714 |
| 15 | - | - | + | - |
| 22 | - | - | - | - |
| 23 | - | - | - | - |
| 24 | +/- | - | - | - |
| 25 | +/- | - | - | - |
| 32 | +/- | + | + | - |
| 36 | - | - | - | - |
| 46 | - | - | - | - |
| 50 | - | - | - | - |
| 65 | - | - | - | - |
| 70 | - | + | + | - |
| 71 | - | - | + | - |
| 75 | - | - | - | - |
| 89 | - | + + | - | + |
| 95 | - | + + | + + | + |
| 100 | - | + + | - | - |
| 102 | - | - | - | - |
| 108 | - | - | - | - |
| 130 | - | - | + | - |
| 137 | - | - | - | - |
| LG | + | + + | + + + | + |
| 301060 | - | + + | + + + | + |
| PB3178 | + + | + | + + + | + |
| PB3180 | + + | + | + + + | + + |
| 300423 | + + | - | + + + | + |
| % POSITIVE | 16 | 36 | 44 | 27 |

* ALL SAMPLES SHOWED THE PRESENCE OF ANTIBODIES TO HEPATITIS C VIRUS BY EIA
AS WELL AS WESTERN SLOT ANALYSIS.

## Claims
**Claims for the following Contracting States : AT, BE, CH, LI, DE, FR, GB, IT, NL, SE**

1. An assay method for identifying the presence of an antibody immunologically reactive with an HCV antigen in a fluid sample comprising:
   contacting the sample with a polypeptide containing at least one epitope of an HCV antigen selected from the group consisting of p1, p35, p99, p1192, p1223, p1684, p1694, p1866, p1899, p380, p380.LG, p447, p607, p643a, p643b, p666, p691 and p2302 under conditions suitable for complexing the antibody with the polypeptide; and detecting the antibody-polypeptide complex.

2. The assay method of Claim 1 wherein the antigen is p1866, p380, p380.LG, p643b, p666, or p2302.

3. A combination assay method for detecting the presence of an antibody immunologically reactive with an HCV antigen in a fluid sample comprising:
   contacting the sample with a solid support having commonly bound recombinant polypeptide C100-3 and a polypeptide selected from the group consisting of p1, p35, p99, p1192, p1223, p1684, p1694, p1866, p1899, p380, p380.LG, p447, p607, p643a, p643b, p666, p691 and p2302 under conditions suitable for complexing the antibody with the polypeptide; and detecting the antibody-polypeptide complex.

EP 0 445 423 B1

4. A confirmatory assay method for identifying the presence of an antibody in a fluid sample immunologically reactive with an HCV antigen comprising:

contacting a first aliquot of the sample with a first recombinant polypeptide C100-3 which contains at least one epitope of an HCV antigen under conditions suitable for complexing the antibody with the polypeptide and detecting a first antibody-antigen complex;

contacting a second aliquot of the sample with one or more polypeptides selected from the group consisting of p1, p35, p99, p1192, p1223, p1684, p1689, p1694, p1866, p1899, p380, p380.LG, p447, p607, p643a, p643b, p666, p691 and p2302 under conditions suitable to form a second antibody-antigen complex; and

detecting the second antibody-antigen complex;

provided that assay methods employing p1689 alone to form said second antibody-antigen complex are excluded.

5. The assay method of Claim 4 wherein the second antigen is p1684, p1694, p380, p380.LG, p643b, p666, or p2302.

6. An immunodot assay method for identifying the presence of an antibody immunologically reactive with an HCV antigen in a fluid sample comprising:

concurrently contacting the sample with at least two polypeptides each containing distinct epitopes of an HCV antigen under conditions suitable for complexing the antibody with the polypeptides and wherein the antibody-polypeptide is detected by reacting the complex with color-producing reagents,

said polypeptides being selected from the group consisting of p1, p35, p99, p1192, p1223, p1684, p1689, p1694, p1866, p1899, p380, p380.LG, p447, p607, p643a, p643b, p666, p691, p2302 and C100-3 bound to a solid support,

provided that assay methods employing only p1689 and C100-3 are excluded.

7. A competition assay method for identifying the presence of an antibody immunologically reactive with an HCV antigen in a fluid sample comprising preparing first and second immunologically equivalent aliquots of the sample, contacting the first aliquot with solid support containing a bound polypeptide which contains at least one epitope of an HCV antigen under conditions suitable for complexing with the antibody to form a detectable antibody-polypeptide complex and contacting the second aliquot firstly with unbound polypeptide and then with the solid support containing the bound polypeptide,

said polypeptide being selected from the group consisting of p1, p35, p99, p1192, p1223, p1684, p1694, p1866, p1899, p380, p380.LG, p447, p607, p643a, p643b, p666, p691, and p2302.

8. An immunoassay kit comprising:

a polypeptide containing at least one epitope of an HCV antigen selected from the group consisting of p1, p35, p99, p1192, p1223, p1684, p1694, p1866, p1899, p380, p380.LG, p447, p607, p643a, p643b, p666, p691, and p2302;

one or more sample preparation reagents; and

one or more detection and signal producing reagents.

9. The polypeptide p1866.

10. The polypeptides p1, p35, and p99.

11. The polypeptide p380.LG.

12. The polypeptide p2302.

**Claims for the following Contracting State : ES**

1. An assay method for identifying the presence of an antibody immunologically reactive with an HCV antigen in a fluid sample comprising:

contacting the sample with a polypeptide containing at least one epitope of an HCV antigen selected from the group consisting of p1, p35, p99, p1192, p1223, p1684, p1694, p1866, p1899, p380, p380.LG, p447, p607, p643a, p643b, p666, p691 and p2302 under conditions suitable for complexing the antibody with the polypeptide; and detecting the antibody-polypeptide complex.

29

2. The assay method of Claim 1 wherein the antigen is p1866, p380, p380.LG, p643b, p666, or p2302.

3. A combination assay method for detecting the presence of an antibody immunologically reactive with an HCV antigen in a fluid sample comprising:

contacting the sample with a solid support having commonly bound recombinant polypeptide C100-3 and a polypeptide selected from the group consisting of p1, p35, p99, p1192, p1223, p1684, p1694, p1866, p1899, p380, p380.LG, p447, p607, p643a, p643b, p666, p691 and p2302 under conditions suitable for complexing the antibody with the polypeptide; and detecting the antibody-polypeptide complex.

4. A confirmatory assay method for identifying the presence of an antibody in a fluid sample immunologically reactive with an HCV antigen comprising:

contacting a first aliquot of the sample with a first recombinant polypeptide C100-3 which contains at least one epitope of an HCV antigen under conditions suitable for complexing the antibody with the polypeptide and detecting a first antibody-antigen complex;

contacting a second aliquot of the sample with one or more polypeptides selected from the group consisting of p1, p35, p99, p1192, p1223, p1684, p1689, p1694, p1866, p1899, p380, p380.LG, p447, p607, p643a, p643b, p666, p691 and p2302 under conditions suitable to form a second antibody-antigen complex; and

detecting the second antibody-antigen complex;

provided that assay methods employing p1689 alone to form said second antibody-antigen complex are excluded.

5. The assay method of Claim 4 wherein the second antigen is p1684, p1694, p380, p380.LG, p643b, p666, or p2302.

6. An immunodot assay method for identifying the presence of an antibody immunologically reactive with an HCV antigen in a fluid sample comprising:

concurrently contacting the sample with at least two polypeptides each containing distinct epitopes of an HCV antigen under conditions suitable for complexing the antibody with the polypeptides and wherein the antibody-polypeptide is detected by reacting the complex with color-producing reagents,

said polypeptides being selected from the group consisting of p1, p35, p99, p1192, p1223, p1684, p1689, p1694, p1866, p1899, p380, p380.LG, p447, p607, p643a, p643b, p666, p691, p2302 and C100-3 bound to a solid support,

provided that assay methods employing only p1689 and C100-3 are excluded.

7. A competition assay method for identifying the presence of an antibody immunologically reactive with an HCV antigen in a fluid sample comprising preparing first and second immunologically equivalent aliquots of the sample, contacting the first aliquot with solid support containing a bound polypeptide which contains at least one epitope of an HCV antigen under conditions suitable for complexing with the antibody to form a detectable antibody-polypeptide complex and contacting the second aliquot firstly with unbound polypeptide and then with the solid support containing the bound polypeptide,

said polypeptide being selected from the group consisting of p1, p35, p99, p1192, p1223, p1684, p1694, p1866, p1899, p380, p380.LG, p447, p607, p643a, p643b, p666, p691, and p2302.

8. An immunoassay kit comprising:

a polypeptide containing at least one epitope of an HCV antigen selected from the group consisting of p1, p35, p99, p1192, p1223, p1684, p1694, p1866, p1899, p380, p380.LG, p447, p607, p643a, p643b, p666, p691, and p2302;

one or more sample preparation reagents; and

one or more detection and signal producing reagents.

9. An immunoassay kit comprising:

a solid support having commonly bound recombinant polypeptide C100-3 and a polypeptide containing at least one epitope of an HCV antigen selected from the group consisting of p1, p35, p99, p1192, p1223, p1684, p1694, p1866, p1899, p380, p380.LG, p447, p607, p643a, p643b, p666, p691 and p2302;

one or more sample preparation reagents; and

one or more detection and signal producing reagents.

10. An immunoassay kit comprising:

recombinant polypeptide C100-3 bound to a solid support;

one or more polypeptides containing at least one epitope of an HCV antigen bound to a solid support selected from the group consisting of p1, p35, p99, p1192, p1223, p1684, p1689, p1694, p1866, p1899, p380, p380.LG, p447, p607, p643a, p643b, p666, p691 and p2302, provided that the kit does not contain p1689 and C100-3 as the only polypeptides;

one or more sample preparation reagents; and

one or more detection and signal producing reagents.

11. An immunoassay kit comprising:

recombinant polypeptide C100-3 bound to a solid support;

one or more polypeptides containing at least one epitope of an HCV antigen bound to the same solid support as the polypeptide C100-3 selected from the group consisting of p1, p35, p99, p1192, p1223, p1684, p1689, p1694, p1866, p1899, p380, p380.LG, p447, p607, p643a, p643b, p666, p691 and p2302, provided that the kit does not contain p1689 and C100-3 as the only polypeptides;

one or more sample preparation reagents; and

one or more detection and signal producing reagents.

12. An immunoassay kit comprising:

a test cartridge comprising at least two polypeptides each containing distinct epitopes of an HCV antigen, said polypeptides being selected from the group consisting of p1, p35, p99, p1192, p1223, p1684, p1689, p1694, p1866, p1899, p380, p380.LG, p447, p607, p643a, p643b, p666, p691, p2302 and C100-3 bound to a solid support, provided that the test cartridge does not contain p1689 and C100-3 as the only polypeptides;

one or more sample preparation reagents; and

one or more detection and signal producing reagents.

13. An immunoassay kit comprising:

a solid support containing a bound polypeptide containing at least one epitope of an HCV antigen;

unbound polypeptide containing at least one epitope of an HCV antigen;

one or more sample preparation reagents; and

one or more detection and signal producing reagents;

said polypeptide being selected from the group consisting of p1, p35, p99, p1192, p1223, p1684, p1694, p1866, p1899, p380, p380.LG, p447, p607, p643a, p643b, p666, p691, and p2302.

**Patentansprüche**

**Patentansprüche für folgende Vertragsstaaten : AT, BE, CH, LI, DE, FR, GB, IT, NL, SE**

1. Assayverfahren zur Identifizierung der Anwesenheit eines Antikörpers in einer flüssigen Probe, der immunologisch gegenüber einem HCV-Antigen reaktiv ist, folgendes umfassend:

in-Kontakt-Bringen der Probe mit einem Polypeptid, das wenigstens ein Epitop eines HCV-Antigens enthält, das aus der Gruppe gewählt ist, die aus p1, p35, p99, p1192, p1223, p1684, p1694, p1866, p1899, p380, p380.LG, p447, p607, p643a, p643b, p666, p691 und p2302 besteht, unter Bedingungen, die zur Komplexierung des Antikörpers mit dem Polypeptid geeignet sind; und Nachweis des Antikörper-Polypeptidkomplexes.

2. Assayverfahren nach Anspruch 1, wobei das Antigen p1866, p380, p380.LG, p643b, p666 oder p2302 ist.

3. Kombinationsassayverfahren zum Nachweis der Anwesenheit eines Antikörpers, der immunologisch gegenüber einem HCV-Antigen in einer flüssigen Probe reaktiv ist, folgendes umfassend:

in-Kontakt-Bringen der Probe mit einem festen Träger, der herkömmlich gebunden rekombinantes Polypeptid C100-3 und ein Polypeptid aufweist, das aus der Gruppe bestehend aus p1, p35, p99, p1192, p1223, p1684, p1694, p1866, p1899, p380, p380.LG, p447, p607, p643a, p643b, p666, p691 und p2302 gewählt ist, unter Bedingungen, die zur Komplexierung des Antikörpers mit dem Polypeptid geeignet sind; und Nachweis des Antikörper-Polypeptidkomplexes.

**4.** Bestätigungsassayverfahren zur Identifizierung der Anwesenheit eines Antikörpers in einer flüssigen Probe, der gegenüber einem HCV-Antigen immunologisch reaktiv ist, folgendes umfassend:

in-Kontakt-Bringen eines ersten aliquoten Anteils der Probe mit einem ersten rekombinanten Polypeptid C100-3, das wenigstens ein Epitop eines HCV-Antigens enthält, unter Bedingungen, die zur Komplexierung des Antikörpers mit dem Polypeptid und zum Nachweis eines ersten Antikörper-Antigenkomplexes geeignet sind;

in-Kontakt-Bringen eines zweiten aliquoten Anteils der Probe mit einem oder mehreren Polypeptiden, die aus der Gruppe gewählt sind, die aus p1, p35, p99, p1192, p1223, p1684, p1689, p1694, p1866, p1899, p380, p380.LG, p447, p607, p643a, p643b, p666, p691 und p2302 besteht, unter Bedingungen, die zur Bildung eines zweiten Antikörper-Antigenkomplexes geeignet sind; und

Nachweis des zweiten Antikörper-Antigenkomplexes;

vorausgesetzt, daß Assayverfahren, die p1689 alleine zur Bildung des zweiten Antikörper-Antigenkomplexes verwenden, ausgeschlossen sind.

**5.** Assayverfahren nach Anspruch 4, wobei das zweite Antigen p1684, p1694, p380, p380.LG, p643b, p666 oder p2302 ist.

**6.** Immuntüpfel-Assayverfahren zur Identifizierung der Anwesenheit eines Antikörpers in einer flüssigen Probe, der gegenüber einem HCV-Antigen immunologisch reaktiv ist, folgendes umfassend:

Konkurrierendes in-Kontakt-Bringen der Probe mit wenigstens zwei Polypeptiden, die beide verschiedene Epitope eines HCV-Antigens enthalten, unter Bedingungen, die zur Komplexierung des Antikörpers mit den Polypeptiden geeignet sind, und wobei der Antikörper-Polypeptidkomplex durch Umsetzung des Komplexes mit farbbildenden Reagenzien nachgewiesen wird,

wobei die Polypeptide aus der Gruppe gewählt sind, die aus p1, p35, p99, p1192, p1223, p1684, p1689, p1694, p1866, p1899, p380, p380.LG, p447, p607, p643a, p643b, p666, p691, p2302 und C100-3 besteht, und an einen festen Träger gebunden sind,

vorausgesetzt, daß Assayverfahren, die nur p1689 und C100-3 verwenden, ausgeschlossen sind.

**7.** Konkurrenzassayverfahren zur Identifizierung der Anwesenheit eines Antikörpers in einer flüssigen Probe, der gegenüber einem HCV-Antigen immunologisch reaktiv ist, umfassend die Herstellung eines ersten und eines zweiten immunologisch äquivalenten aliquoten Anteils der Probe, das in-Kontakt-Bringen des ersten aliquoten Anteils mit einem festen Träger, der ein gebundenes Polypeptid aufweist, das wenigstens ein Epitop eines HCV-Antigens enthält, unter Bedingungen, die für die Komplexierung mit dem Antikörper unter Bildung eines nachweisbaren Antikörper-Polypeptidkomplexes geeignet sind, und das in-Kontakt-Bringen des zweiten aliquoten Anteils zunächst mit ungebundenem Polypeptid und dann mit dem festen Träger, der das gebundene Polypeptid aufweist,

wobei das Polypeptid aus der Gruppe gewählt ist, die aus p1, p35, p99, p1192, p1223, p1684, p1694, p1866, p1899, p380, p380.LG, p447, p607, p643a, p643b, p666, p691 und p2302 besteht.

**8.** Immunoassaykit, folgendes umfassend:

ein Polypeptid, das wenigstens ein Epitop eines HCV-Antigens enthält, das aus der Gruppe gewählt ist, die aus p1, p35, p99, p1192, p1223, p1684, p1694, p1866, p1899, p380, p380.LG, p447, p607, p643a, p643b, p666, p691 und p2302 besteht;

eines oder mehrere Probenvorbehandlungsreagenzien; und

eines oder mehrere Nachweis- und Signalerzeugungsreagenzien.

**9.** Das Polypeptid p1866.

**10.** Die Polypeptide p1, p35 und p99.

**11.** Das Polypeptid p380.LG.

**12.** Das Polypeptid p2302.

**Patentansprüche für folgenden Vertragsstaat : ES**

**1.** Assayverfahren zur Identifizierung der Anwesenheit eines Antikörpers in einer flüssigen Probe, der immunologisch gegenüber einem HCV-Antigen reaktiv ist, folgendes umfassend:

in-Kontakt-Bringen der Probe mit einem Polypeptid, das wenigstens ein Epitop eines HCV-Antigens enthält, das aus der Gruppe gewählt ist, die aus p1, p35, p99, p1192, p1223, p1684, p1694, p1866, p1899, p380, p380.LG, p447, p607, p643a, p643b, p666, p691 und p2302 besteht, unter Bedingungen, die zur Komplexierung des Antikörpers mit dem Polypeptid geeignet sind; und Nachweis des Antikörper-Polypeptidkomplexes.

2. Assayverfahren nach Anspruch 1, wobei das Antigen p1866, p380, p380.LG, p643b, p666 oder p2302 ist.

3. Kombinationsassayverfahren zum Nachweis der Anwesenheit eines Antikörpers in einer flüssigen Probe, der immunologisch gegenüber einem HCV-Antigen reaktiv ist, folgendes umfassend:

in-Kontakt-Bringen der Probe mit einem festen Träger, der herkömmlich gebunden rekombinantes Polypeptid C100-3 und ein Polypeptid aufweist, das aus der Gruppe bestehend aus p1, p35, p99, p1192, p1223, p1684, p1694, p1866, p1899, p380, p380.LG, p447, p607, p643a, p643b, p666, p691 und p2302 gewählt ist, unter Bedingungen, die zur Komplexierung des Antikörpers mit dem Polypeptid geeignet sind; und Nachweis des Antikörper-Polypeptidkomplexes.

4. Bestätigungsassayverfahren zur Identifizierung der Anwesenheit eines Antikörpers in einer flüssigen Probe, der gegenüber einem HCV-Antigen immunologisch reaktiv ist, folgendes umfassend:

in-Kontakt-Bringen eines ersten aliquoten Anteils der Probe mit einem ersten rekombinanten Polypeptid C100-3, das wenigstens ein Epitop eines HCV-Antigens enthält, unter Bedingungen, die zur Komplexierung des Antikörpers mit dem Polypeptid und zum Nachweis eines ersten Antikörper-Antigenkomplexes geeignet sind;

in-Kontakt-Bringen eines zweiten aliquoten Anteils der Probe mit einem oder mehreren Polypeptiden, die aus der Gruppe, gewählt sind, die aus p1, p35, p99, p1192, p1223, p1684, p1689, p1694, p1866, p1899, p380, p380.LG, p447, p607, p643a, p643b, p666, p691 und p2302 besteht, unter Bedingungen, die zur Bildung eines zweiten Antikörper-Antigenkomplexes geeignet sind; und

Nachweis des zweiten Antikörper-Antigenkomplexes;

vorausgesetzt, daß Assayverfahren, die p1689 alleine zur Bildung des zweiten Antikörper-Antigenkomplexes verwenden, ausgeschlossen sind.

5. Assayverfahren nach Anspruch 4, wobei das zweite Antigen p1684, p1694, p380, p380.LG, p643b, p666 oder p2302 ist.

6. Immuntüpfel-Assayverfahren zur Identifizierung der Anwesenheit eines Antikörpers in einer flüssigen Probe, der gegenüber einem HCV-Antigen immunologisch reaktiv ist, folgendes umfassend:

Konkurrierendes in-Kontakt-Bringen der Probe mit wenigstens zwei Polypeptiden, die beide definierte Epitope eines HCV-Antigens enthalten, unter Bedingungen, die zur Komplexierung des Antikörpers mit den Polypeptiden geeignet sind, und wobei der Antikörper-Polypeptidkomplex durch Umsetzung des Komplexes mit farbbildenden Reagenzien nachgewiesen wird,

wobei die Polypeptide aus der Gruppe gewählt sind, die aus p1, p35, p99, p1192, p1223, p1684, p1689, p1694, p1866, p1899, p380, p380.LG, p447, p607, p643a, p643b, p666, p691, p2302 und C100-3 besteht, und an einen festen Träger gebunden sind,

vorausgesetzt, daß Assayverfahren, die nur p1689 und C100-3 verwenden, ausgeschlossen sind.

7. Konkurrenzassayverfahren zur Identifizierung der Anwesenheit eines Antikörpers in einer flüssigen Probe, der gegenüber einem HCV-Antigen immunologisch reaktiv ist, umfassend die herstellung eines ersten und eines zweiten immunologisch äquivalenten aliquoten Anteils der Probe, das in-Kontakt-Bringen des ersten aliquoten Anteils mit einem festen Träger, der ein gebundenes Polypeptid aufweist, das wenigstens ein Epitop eines HCV-Antigens enthält, unter Bedingungen, die für die Komplexierung mit dem Antikörper unter Bildung eines nachweisbaren Antikörper-Polypeptidkomplexes geeignet sind, und das in-Kontakt-Bringen des zweiten aliquoten Anteils zunächst mit ungebundenem Polypeptid und dann mit dem festen Träger, der das gebundene Polypeptid aufweist,

wobei das Polypeptid aus der Gruppe gewählt ist, die aus p1, p35, p99, p1192, p1223, p1684, p1694, p1866, p1899, p380, p380.LG, p447, p607, p643a, p643b, p666, p691 und p2302 besteht.

8. Immunoassaykit, folgendes umfassend:

ein Polypeptid, das wenigstens ein Epitop eines HCV-Antigens enthält, das aus der Gruppe gewählt ist, die aus p1, p35, p99, p1192, p1223, p1684, p1694, p1866, p1899, p380, p380.LG, p447, p607, p643a, p643b, p666, p691 und p2302 besteht;
eines oder mehrere Probenvorbehandlungsreagenzien; und
eines oder mehrere Nachweis- und Signalerzeugungsreagenzien.

9. Immunoassaykit, folgendes umfassend:
einen festen Träger, der herkömmlich gebunden rekombinantes Polypeptid C100-3 und ein Polypeptid aufweist, das wenigstens ein Epitop eines HCV-Antigens enthält, das aus der Gruppe bestehend aus p1, p35, p99, p1192, p1223, p1684, p1694, p1866, p1899, p380, p380.LG, p447, p607, p643a, p643b, p666, p691 und p2302 gewählt ist;
eines oder mehrere Probenvorbehandlungsreagenzien; und
eines oder mehrere Nachweis- und Signalerzeugungsreagenzien.

10. Immunoassaykit, folgendes umfassend:
rekombinantes Polypeptid C100-3, das an einen festen Träger gebunden ist;
eines oder mehrere an einen festen Träger gebundene Polypeptide, die wenigstens ein Epitop eines HCV-Antigens enthalten, die aus der Gruppe gewählt sind, die aus p1, p35, p99, p1192, p1223, p1684, p1689, p1694, p1866, p1899, p380, p380.LG, p447, p607, p643a, p643b, p666, p691 und p2302 besteht, vorausgesetzt, daß das Kit p1689 und C100-3 nicht als die einzigen Polypeptide enthält;
eines oder mehrere Probenvorbehandlungsreagenzien; und
eines oder mehrere Nachweis- und Signalerzeugungsreagenzien.

11. Immunoassaykit, folgendes umfassend:
rekombinantes Polypeptid C100-3, das an einen festen Täger gebunden ist;
eines oder mehrere Polypeptide, die wenigstens ein Epitop eines HCV-Antigens an den selben festen Träger wie das Polypeptid C100-3 gebunden aufweisen, die aus der Gruppe bestehend aus p1, p35, p99, p1192, p1223, p1684, p1689, p1694, p1866, p1899, p380, p380.LG, p447, p607, p643a, p643b, p666, p691 und p2302 gewählt sind, vorausgesetzt, daß das Kit p1689 und C100-3 nicht als die einzigen Polypeptide enthält;
ein oder mehrere Probenvorbehandlungsreagenzien; und
ein oder mehrere Nachweis- und Signalerzeugungsreagenzien.

12. Immunoassaykit, folgendes umfassend:
eine Testpatrone, die wenigstens zwei Polypeptide umfaßt, die beide verschiedene Epitope eines HCV-Antigens enthalten, wobei die Polypeptide aus der Gruppe gewählt sind, die aus p1, p35, p99, p1192, p1223, p1684, p1689, p1694, p1866, p1899, p380, p380.LG, p447, p607, p643a, p643b, p666, p691, p2302 und C100-3 besteht, die an einen festen Träger gebunden sind, vorausgesetzt, daß die Testpatrone p1689 und C100-3 nicht als die einzigen Polypeptide enthält;
eines oder mehrere Probenvorbehandlungsreagenzien; und
eines oder mehrere Nachweis- und Signalerzeugungsreagenzien.

13. Immunoassaykit, folgendes umfassend:
einen festen Träger, der ein gebundenes Polypeptid aufweist, das wenigstens ein Epitop eines HCV-Antigens enthält;
ungebundenes Polypeptid, das wenigstens ein Epitop eines HCV-Antigens enthält;
eines oder mehrere Probenvorbehandlungsreagenzien; und
eines oder mehrere Nachweis- und Signalerzeugungsreagenzien;
wobei das Polypeptid aus der Gruppe gewählt ist, die aus p1, p35, p99, p1192, p1223, p1684, p1694, p1866, p1899, p380, p380.LG, p447, p607, p643a, p643b, p666, p691 und p2302 besteht.

**Revendications**
**Revendications pour les Etats contractants suivants : AT, BE, CH, LI, DE, FR, GB, IT, NL, SE**

1. Procédé de dosage pour identifier la présence d'un anticorps immunologiquement réactif avec un antigène de HCV dans un échantillon de liquide, comprenant :
la mise en contact de l'échantillon avec un polypeptide contenant au moins un épitope d'un antigène de HCV choisi dans le groupe consistant en p1, p35, p99, p1192, p1223, p1684, p1694, p1866, p1899, p380, p380.LG, p447, p607, p643a, p643b, p666, p691 et p2302 dans des conditions appropriées pour la complexation de l'anticorps avec le polypeptide, et la détection du complexe anticorps-polypeptide.

2. Procédé de dosage selon la revendication 1, dans lequel l'antigène est p1866, p380, p380.LG, p643b, p666 ou p2302.

3. Procédé de dosage en combinaison pour détecter la présence d'un anticorps immunologiquement réactif avec un antigène de HCV dans un échantillon de liquide, comprenant :
la mise en contact de l'échantillon avec un support solide auquel sont liés simultanément le polypeptide recombiné C100-3 et un polypeptide choisi dans le groupe consistant en p1, p35, p99, p1192, p1223, p1684, p1694, p1866, p1899, p380, p380.LG, p447, p607, p643a, p643b, p666, p691 et p2302 dans des conditions appropriées pour la complexation de l'anticorps avec le polypeptide, et la détection du complexe anticorps-polypeptide.

4. Procédé de dosage de confirmation pour identifier la présence d'un anticorps dans un échantillon de liquide immunologiquement réactif avec un anti-gène de HCV, comprenant :
la mise en contact d'une première portion aliquote de l'échantillon avec un premier polypeptide recombiné C100-3 qui contient au moins un épitope d'un antigène de HCV dans des conditions appropriées pour la complexation de l'anti-corps avec le polypeptide et la détection d'un premier complexe anticorps-antigène,
la mise en contact d'une seconde portion aliquote de l'échantillon avec un ou plusieurs polypeptides choisis dans le groupe consistant en p1, p35, p99, p1192, p1223, p1684, p1689, p1694, p1866, p1899, p380, p380.LG, p447, p607, p643a, p643b, p666, p691 et p2302 dans des conditions appropriées pour former un second complexe anticorps-antigène, et
la détection du second complexe anticorps-antigène,
à condition que les procédés de dosage employant p1689 seul pour former ledit second complexe anticorps-antigène soient exclus.

5. Procédé de dosage selon la revendication 4, dans lequel le second antigène est p1684, p1694, p380, p380.LG, p643b, p666 ou p2302.

6. Procédé de dosage par immunotaches pour identifier la présence d'un anticorps immunologiquement réactif avec un antigène de HCV dans un échantillon de liquide, comprenant :
la mise en contact simultanée de l'échantillon avec au moins deux polypeptides contenant chacun des épitopes distincts d'un antigène de HCV dans des conditions appropriées pour la complexation de l'anticorps avec les polypeptides et dans lequel l'anticorps-polypeptide est détecté par réaction du complexe avec des réactifs producteurs de couleur,
lesdits polypeptides étant choisis dans le groupe consistant en p1, p35, p99, p1192, p1223, p1684, p1689, p1694, p1866, p1899, p380, p380.LG, p447, p607, p643a, p643b, p666, p691, p2302 et C100-3 liés à un support solide, à condition que les procédés de dosage employant seulement p1689 et C100-3 soient exclus.

7. Procédé de dosage par compétition pour identifier la présence d'un anticorps immunologiquement réactif avec un antigène de HCV dans un échantillon de liquide, comprenant la préparation de première et seconde portions aliquotes immunologiquement équivalentes de l'échantillon, la mise en contact de la première portion aliquote avec un support solide contenant un polypeptide lié qui contient au moins un épitope d'un antigène de HCV dans des conditions appropriées pour la complexation avec l'anticorps pour former un complexe anticorps-polypeptide détectable et la mise en contact de la seconde portion aliquote tout d'abord avec un polypeptide non lié puis avec le support solide contenant le polypeptide lié, ledit polypeptide étant choisi dans le groupe consistant en p1, p35, p99, p1192, p1223, p1684, p1694, p1866, p1899, p380, p380.LG, p447, p607, p643a, p643b, p666, p691 et p2302.

8. Trousse d'immunodosage comprenant :
un polypeptide contenant au moins un épitope d'un antigène de HCV choisi dans le groupe consistant en p1, p35, p99, p1192, p1223, p1684, p1694, p1866, p1899, p380, p380.LG, p447, p607, p643a, p643b, p666, p691 et p2302,
un ou plusieurs réactifs de préparation d'échantillon, et
un ou plusieurs réactifs de détection et de production de signal.

**9.** Polypeptide p1866.

**10.** Polypeptides p1, p35 et p99.

**11.** Polypeptide p380.LG.

**12.** Polypeptide p2302.

**Revendications pour l'Etat contractant suivant : ES**

**1.** Procédé de dosage pour identifier la présence d'un anticorps immunologiquement réactif avec un antigène de HCV dans un échantillon de liquide, comprenant :

la mise en contact de l'échantillon avec un polypeptide contenant au moins un épitope d'un antigène de HCV choisi dans le groupe consistant en p1, p35, p99, p1192, p1223, p1684, p1694, p1866, p1899, p380, p380.LG, p447, p607, p643a, p643b, p666, p691 et p2302 dans des conditions appropriées pour la complexation de l'anticorps avec le polypeptide, et la détection du complexe anticorps-polypeptide.

**2.** Procédé de dosage selon la revendication 1, dans lequel l'antigène est p1866, p380, p380.LG, p643b, p666 ou p2302.

**3.** Procédé de dosage en combinaison pour détecter la présence d'un anticorps immunologiquement réactif avec un antigène de HCV dans un échantillon de liquide, comprenant :

la mise en contact de l'échantillon avec un support solide auquel sont liés simultanément le polypeptide recombiné C100-3 et un polypeptide choisi dans le groupe consistant en p1, p35, p99, p1192, p1223, p1684, p1694, p1866, p1899, p380, p380.LG, p447, p607, p643a, p643b, p666, p691 et p2302 dans des conditions appropriées pour la complexation de l'anticorps avec le polypeptide, et la détection du complexe anticorps-polypeptide.

**4.** Procédé de dosage de confirmation pour identifier la présence d'un anticorps dans un échantillon de liquide immunologiquement réactif avec un antigène de HCV, comprenant :

la mise en contact d'une première portion aliquote de l'échantillon avec un premier polypeptide recombiné C100-3 qui contient au moins un épitope d'un antigène de HCV dans des conditions appropriées pour la complexation de l'anticorps avec le polypeptide et la détection d'un premier complexe anticorps-antigène,

la mise en contact d'une seconde portion aliquote de l'échantillon avec un ou plusieurs polypeptides choisis dans le groupe consistant en p1, p35, p99, p1192, p1223, p1684, p1689, p1694, p1866, p1899, p380, p380.LG, p447, p607, p643a, p643b, p666, p691 et p2302 dans des conditions appropriées pour former un second complexe anticorps-antigène, et

la détection du second complexe anticorps-antigène,

à condition que les procédés de dosage employant p1689 seul pour former ledit second complexe anticorps-antigène soient exclus.

**5.** Procédé de dosage selon la revendication 4, dans lequel le second antigène est p1684, p1694, p380, p380.LG, p643b, p666 ou p2302.

**6.** Procédé de dosage par immunotaches pour identifier la présence d'un anticorps immunologiquement réactif avec un antigène de HCV dans un échantillon de liquide, comprenant :

la mise en contact simultanée de l'échantillon avec au moins deux polypeptides contenant chacun des épitopes distincts d'un antigène de HCV dans des conditions appropriées pour la complexation de l'anticorps avec les polypeptides et dans lequel l'anticorps-polypeptide est détecté par réaction du complexe avec des réactifs producteurs de couleur,

lesdits polypeptides étant choisis dans le groupe consistant en p1, p35, p99, p1192, p1223, p1684, p1689, p1694, p1866, p1899, p380, p380.LG, p447, p607, p643a, p643b, p666, p691, p2302 et C100-3 liés à un support solide, à condition que les procédés de dosage employant seulement p1689 et C100-3 soient exclus.

7. Procédé de dosage par compétition pour identifier la présence d'un anticorps immunologiquement réactif avec un antigène de HCV dans un échantillon de liquide, comprenant la préparation de première et seconde portions aliquotes immunologiquement équivalentes de l'échantillon, la mise en contact de la première portion aliquote avec un support solide contenant un polypeptide lié qui contient au moins un épitope d'un antigène de HCV dans des conditions appropriées pour la complexation avec l'anticorps pour former un complexe anticorps-polypeptide détectable et la mise en contact de la seconde portion aliquote tout d'abord avec un polypeptide non lié puis avec le support solide contenant le polypeptide lié, ledit polypeptide étant choisi dans le groupe consistant en p1, p35, p99, p1192, p1223, p1684, p1694, p1866, p1899, p380, p380.LG, p447, p607, p643a, p643b, p666, p691 et p2302.

8. Trousse d'immunodosage comprenant :
un polypeptide contenant au moins un épitope d'un antigène de HCV choisi dans le groupe consistant en p1, p35, p99, p1192, p1223, p1684, p1694, p1866, p1899, p380, p380.LG, p447, p607, p643a, p643b, p666, p691 et p2302,
un ou plusieurs réactifs de préparation d'échantillon, et
un ou plusieurs réactifs de détection et de production de signal.

9. Trousse d'immunodosage comprenant :
un support solide auquel sont liés simultanément le polypeptide recombiné C100-3 et un polypeptide contenant au moins un épitope d'un antigène de HCV choisi dans le groupe consistant en p1, p35, p99, p1192, p1223, p1684, p1694, p1866, p1899, p380, p380.LG, p447, p607, p643a, p643b, p666, p691 et p2302,
un ou plusieurs réactifs de préparation d'échantillon, et
un ou plusieurs réactifs de détection et de production de signal.

10. Trousse d'immunodosage comprenant :
le polypeptide recombiné C100-3 lié à un support solide,
un ou plusieurs polypeptides contenant au moins un épitope d'un anti-gène de HCV lié à un support solide choisi dans le groupe consistant en p1, p35, p99, p1192, p1223, p1684, p1689, p1694, p1866, p1899, p380, p380.LG, p447, p607, p643a, p643b, p666, p691 et p2302, à condition que la trousse ne contienne pas p1689 et C100-3 comme seuls polypeptides,
un ou plusieurs réactifs de préparation d'échantillon, et
un ou plusieurs réactifs de détection et de production de signal.

11. Trousse d'immunodosage comprenant :
le polypeptide recombiné C100-3 lié à un support solide,
un ou plusieurs polypeptides contenant au moins un épitope d'un anti-gène de HCV lié au même support solide que le polypeptide C100-3 choisi dans le groupe consistant en p1, p35, p99, p1192, p1223, p1684, p1689, p1694, p1866, p1899, p380, p380.LG, p447, p607, p643a, p643b, p666, p691 et p2302, à condition que la trousse ne contienne pas p1689 et C100-3 comme seuls polypeptides,
un ou plusieurs réactifs de préparation d'échantillon, et
un ou plusieurs réactifs de détection et de production de signal.

12. Trousse d'immunodosage comprenant :
une cartouche de test comprenant au moins deux polypeptides contenant chacun des épitopes distincts d'un antigène de HCV, lesdits polypeptides étant choisis dans le groupe consistant en p1, p35, p99, p1192, p1223, p1684, p1689, p1694, p1866, p1899, p380, p380.LG, p447, p607, p643a, p643b, p666, p691 et p2302, liés à un support solide, à condition que la cartouche de test ne contienne pas p1689 et C100-3 comme seuls polypeptides,
un ou plusieurs réactifs de préparation d'échantillon, et
un ou plusieurs réactifs de détection et de production de signal.

13. Trousse d'immunodosage comprenant :
un support solide contenant un polypeptide lié contenant au moins un épitope d'un antigène de HCV,
un polypeptide non lié contenant au moins un épitope d'un antigène de HCV,
un ou plusieurs réactifs de préparation d'échantillon, et
un ou plusieurs réactifs de détection et de production de signal,

ledit polypeptide étant choisi dans le groupe consistant en p1, p35, p99, p1192, p1223, p1684, p1694, p1866, p1899, p380, p380.LG, p447, p607, p643a, p643b, p666, p691 et p2302.

| CORE | ENVELOPE | NS1 | NS 2 | NS 3 | NS 4 | NS 5 |

P1   1–75 (75 AA)

P35  35–75 (41 AA)

FIG. 1a

EP 0 445 423 B1

FIG. 1b

FIG. 2

EP 0 445 423 B1

FIG. 3a

EP 0 445 423 B1

FIG. 3b

CONTROLS

FIG. 4

FIG. 5